# EUROPEAN PATENT APPLICATION

(11) **EP 3 785 529 A1**
(43) Date of publication of application: **03.03.2021**
(21) Application number: 19193990.9
(22) Date of filing: 28.08.2019
(51) Int. Cl.: A01H 5/10, A01H 6/46, C12N 15/82, C12Q 1/6895, C12C 1/00

(54) **BARLEY PLANTS WITH ALTERED PROTEIN CONTENT IN GRAINS**

(71) Applicant: Carlsberg A/S, 1799 Copenhagen V (DK)
(72) Inventor: KNUDSEN, Søren, 1799 Copenhagen V (DK); OLSEN, Ole, 2300 Copenhagen S (DK); THOMSEN, Hanne Cecilie, 1799 Copenhagen V (DK); PEDAS, Pai Rosager, 1799 Copenhagen V (DK); WENDT, Toni, 1799 Copenhagen V (DK); SKADHAUGE, Birgitte, 1799 Copenhagen V (DK)
(74) Representative: Høiberg P/S

(57) **Abstract**

The present invention relates to barley plants having grains with altered grain protein content due to mutation of the NAC transcription factor gene NAM-1. In particular, the invention relates to barley plants having grains with a grain protein content advantageous for production of barley based beverages, e.g. beer. The invention further relates to methods for production of barley based beverages, as well as to products prepared from the barley plants of the invention.

## Description

### Technical field

The present invention relates to barley plants having grains with altered grain protein content. In particular, the invention relates to barley plants having a grain protein content advantageous for production of barley based beverages, e.g. beer. The invention further relates to methods for production of barley based beverages, as well as to products prepared from the barley plants of the invention.

### Background

The protein content of cereal grains referred to as "grain protein content (GPC) is an important feature in determining cereal quality. It is generally preferred that cereals to be employed in brewing have a relatively low GPC, although it should not be too low. Too much grain protein negatively influences malting, can lead to off flavours in cereal based beverages and high levels of proteins can be problematic for haze formation. For example, some amino acids may act as precursors for Strecker aldehydes, which are considered off-flavours. Furthermore, high protein levels are frequently associated with less starch in the cereal grains. In contrast, free amino nitrogen is needed for growth of yeast during fermentation of cereal based beverages.

The GPC is dependent on growth conditions. Thus, when growing cereal plants in soils with high ammonia levels and/or low humidity, the harvested grains may have a high GPC.

Jamar et al. 2010, describes identification of three haplotypes of the NAM-1 gene in Hordeum. Differences in GPC between investigated H. vulgare varieties were found, but no polymorphisms in the NAM-1 gene were observed. The document further describes a polymorphism leading to the introduction of a premature stop codon in H. bulbosum. H. bulbosum is a wild relative of domestic Hordeum vulgare, but there are significant differences between the species. For example, H. bulbosum has only a moderate feed quality and yield, and typically low starch levels with a different starch composition compared to H. vulgare. Jamar et al. 2010 reports one study describing GPC levels in wild H. bulbosum growing at high altitudes (Arzani et al., 2006). No comparison between GPC of H. vulgare and H. bulbosum grown under similar conditions is provided and thus the study does not allow any conclusions.

Wang et al., 2015 discusses the origin of worldwide cultivated barley with reference to the NAM-1 gene and grain protein content. Wang et al., 2015 finds that two haplotypes both caused by a base mutation at genomic position 544 might have a significant impact on GPC. One of these, named Hap7 carries a G on genomic position 544. Hap7 appears to have a lower GPC than Hap2, which carries a C on genomic position 544. The genomic position 544 is provided relative to the sequence on GenBank accession number DQ869678. This position corresponds to position 304 of the coding sequence given herein as SEQ ID NO:2. The haplotype Hap7 is widespread and found in more than 50% of the investigated barley plants in Europe, United States and Australia. Thus, Hap7 is considered a wild type NAM-1 sequence herein.

### Summary

Barley plants with grains having optimal grain protein content (GPC) are useful in the production of barley/malt based beverages such as beer. The level of GPC in barley grains is dependent on the barley variety, but also on the growth condition of the barley plant. Thus, in order to determine whether the level of GPC is "low" or "high", the level is typically evaluated in comparison with other barley grains from plants grown under similar conditions.

In particular, for cultivation of barley plants under conditions of high levels of ammonia in the soil and/or under dry conditions, barley plants producing grains with reduced grain protein content and desirable.

Low levels of GPC generally allows for a high content of starch in the grains. On the other hand, a certain level of soluble nitrogen (e.g. in form of amino acids in wort are required for efficient fermentation during beer production. Soluble nitrogen levels in wort correlate at least to some extent with the GPC in the cereal plants used for production of said wort. Thus, cereal plants with too low levels of GPCare also not desirable, since they will potentially give too low levels of amino acids in the wort to allow for optimal fermentation conditions.

There is thus a need for the barley plants producing grains, which have an appropriate level of GPC. In particular, there is a need for barley plants with low levels of grain protein content. Such barley plants are in particular useful for cultivation in soils with high ammonia levels and/or low humidity.

The present invention provides a barley plant or a part thereof, wherein said barley plant carries a mutation in the *NAM-1* gene, wherein said mutated *NAM-1* gene encodes a mutant NAM-1 polypeptide, wherein the mutation is one of the following mutations
- a missense mutation in the NAC domain B of HvNAM-1;
- a missense mutation in the NAC domain A of HvNAM-1;
- a missense mutation resulting in a change from a charged amino acid to an uncharged amino acid in the NAC domain C of HvNAM-1; or
- a missense mutation resulting in a change from a Ser to Asn in the C-terminus of HvNAM-1.

In one embodiment the invention provides a barley plant or a part thereof, wherein said barley plant carries a mutation in the *NAM-1* gene, wherein said mutated *NAM-1* gene encodes a mutant NAM-1 polypeptide carrying a missense mutation in the NAC domain B of HvNAM-1. Such barley plants are characterised by a reduced level of GPC compared to wild type barley plants. However, even though the level is reduced compared to wild type barley plants, the level is not reduced too much. Thus, such barley plants are characterised by an adequate level of GPC.

The invention further provides plant products, such as malt, wort or beverages prepared from the barley plants of the invention, as well as methods for producing the barley plants and the plants products.

### Description of Drawings

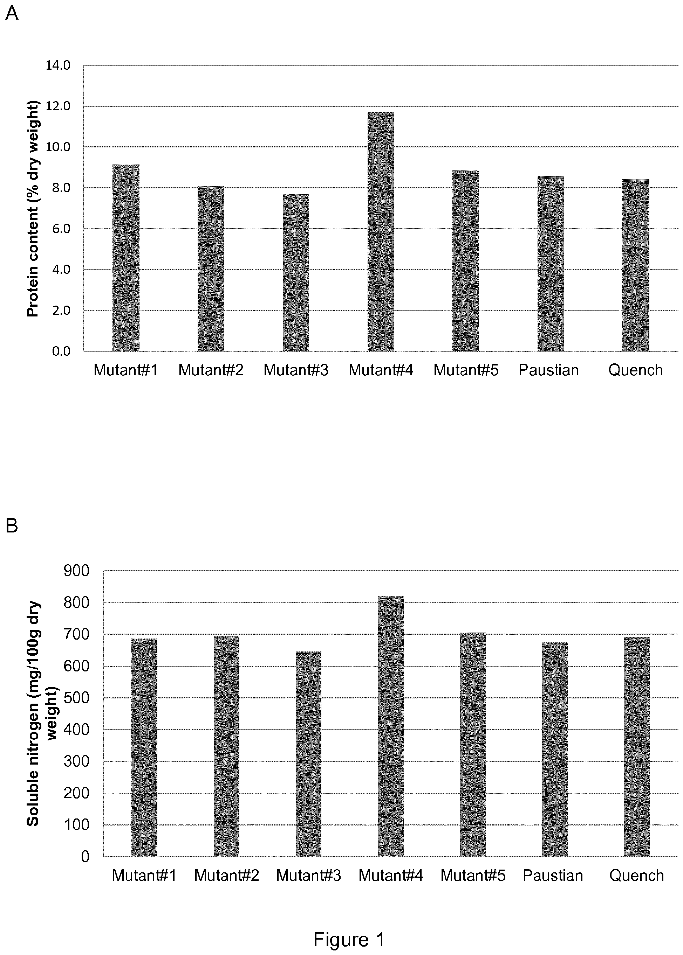

Figure 1. Protein content in kernels in % total dry weight (A) and soluble nitrogen in mg/100g dry weight (B) in malt produced from barley plants mutants 1, 2, 3, 4 and 5 and wild type (WT) cv. Paustian and cv. Quench.

### Detailed description

### Definitions

As used herein, "a" can mean one or more, depending on the context in which it is used.

The term "adjunct" as used herein refers to carbon-rich raw material sources added during preparation of beer. The adjunct may be an ungerminated cereal grain, which may be milled together with the germinated grains prepared according to the invention. The adjunct may also be a syrup, sugar or the like.

The term "amino acid" as used herein refers to a proteinogenic amino acid. Preferably, the proteinogenic amino acids is one of the 20 amino acids encoded by the standard genetic code. The IUPAC one and three letter codes are used to name amino acids.

The term "amino acid corresponding to X" is used herein to describe amino acids of a given polypeptide (e.g. a mutant NAM-1 polypeptide) in relation to amino acids of a reference polypeptide (e.g. NAM-1 of SEQ ID NO:1). Following alignment between said polypeptide and the reference polypeptide, an amino acid is corresponding to X if it is in the same position as X in said alignment.

The term "approximately" when used herein in relation to numerical values preferably means ±10%, more preferably ±5%, yet more preferably ±1%.

The term "barley" in reference to the process of making barley based beverages, such as beer, particularly when used to describe the malting process, means barley kernels. In all other cases, unless otherwise specified, "barley" means the barley plant (*Hordeum vulgare*), including any breeding line or cultivar or variety, whereas part of a barley plant may be any part of a barley plant, for example any tissue or cells.

A "cereal" plant, as defined herein, is a member of the Poaceae plant family, cultivated primarily for their starch-containing seeds or kernels. Cereal plants include, but are not limited to barley (Hordeum vulgare), wheat (Triticum), rice (Oryza), maize (Zea), rye (Secale), oat (Avena), sorghum (Sorghum), and Triticale, a rye-wheat hybrid.

The term "chit" as used herein refers to the embryonic growing bud that is visible during the germination phase of a cereal grain.

The term "charged amino acid" as used herein refers to amino acids with electrically charged side chains. Preferably, the charged amino acid is selected from the group consisting of Arg, His, Lys, Asp and Glu. Negatively charged amino acids are preferably selected from the group consisting of Asp and Glu.

By "encoding" or "encoded", in the context of a specified nucleic acid, is meant comprising the information for translation into the specified protein. A nucleic acid or polynucleotide encoding a protein may comprise non-translated sequences, e.g. introns, within translated regions of the nucleic acid, or may lack such intervening non-translated sequences, e.g. in cDNA. The information by which a protein is encoded is specified by the use of codons.

As used herein, "expression" in the context of nucleic acids is to be understood as the transcription and accumulation of mRNA. "Expression" used in the context of proteins refers to translation of mRNA into a polypeptide.

The term "gene" means the segment of DNA involved in producing a polypeptide chain; it includes regions preceding and following the coding region (promoter and terminator). Furthermore, plant genes generally consist of exons interrupted by introns.

The term "germinated grain" as used herein refers to a grain having developed a visible chit and a visible stem.

The term "grain protein content" as used herein refers to the protein content in grains. Typically, the grain protein content is measured by weight of protein compared to total dry weight of grains. The abbreviation "GPC" is used herein for "grain protein content".

The term "initiation of germination" as used herein refers to the time point at which barley grains with a water content of less than 15% is contacted with sufficient water to initiate germination.

The term "kernel" is defined to comprise the cereal caryopsis, also denoted internal seed. In addition, the kernel may comprise the lemma and palea. In most barley varieties, the lemma and palea adhere to the caryopsis and are a part of the kernel following threshing. However, naked barley varieties also occur. In these, the caryopsis is free of the lemma and palea and threshes out free as in wheat. The terms "kernel" and "grain" are used interchangeably herein.

The term "malting" as used herein refers to a controlled germination of cereal kernels (in particular barley kernels) taking place under controlled environmental conditions. In some embodiments "malting" may further comprise a step of drying said germinated cereal kernels, e.g. by kiln drying.

The term "green malt" as used herein refers germinated cereal kernels, which have not been subjected to a step of kiln drying. In general, said cereal kernels have been germinated under controlled environmental conditions.

The term "kiln dried malt" as used herein refers germinated cereal kernels, which have been dried by kiln drying. In general, said cereal kernels have been germinated under controlled environmental conditions.

"Mashing" is the incubation of milled malt (e.g. green malt or kiln dried malt), and/or ungerminated cereal kernels in water. Mashing is preferably performed at specific temperature(s), and in a specific volume of water.

The term "missense mutation" as used herein refers to a mutation resulting in a change from one amino acid to another.

"Mutations" include deletions, insertions, substitutions, transversions, and point mutations in the coding and noncoding regions of a gene. Deletions may be of the entire gene, or of only a portion of the gene. Point mutations may concern changes of one base pair, and may for result in premature stop codons, frameshift mutations, mutation of a splice site or amino acid substitutions. A gene comprising a mutation may be referred to as a "mutant gene". If said mutant gene encodes a polypeptide with a sequence different to the wild type, said polypeptide may be referred to as a "mutant polypeptide". A mutant polypeptide may comprise an amino acid substitution. The term "amino acid XX at position n has been substituted to amino acid YY" as used herein to means that the amino acid XX of the wild type polypeptide has been replaced by amino acid YY in the mutant polypeptide.

The term "non-polar amino acid" as used herein refers to amino acids with a hydrophobic side chains. Preferably, the non-polar amino acid is selected from the group consisting of Ala, Val, Ile, Leu, Met, Phe, Tyr, Trp and Gly, more preferably from the group consisting of Ala, Val, Ile, Leu, Met, Phe, Trp and Gly.

By the term "plant product" is meant a product resulting from the processing of a plant or plant material. Said plant product may thus, for example, be green malt, kiln dried malt, wort, a fermented or non-fermented beverage, a food, or a feed product.

The term "polar amino acid" as used herein refers to amino acids with polar, uncharged side chains. Preferably, the polar amino acid is selected from the group consisting of Ser, Thr, Asn and Gln.

The term "progeny" as used herein refers to any plant, which has a given plants as one of its ancestors. Progeny not only comprises direct progeny of a given plant, but also progeny after a multitude of generations, for example progeny after up to 100 generations. It may be determined whether a plant is progeny of a given parent plant by determining whether said plant carries the same mutation(s) in the *NAM-1* gene as said parent plant. In addition to the mutation(s) in the *NAM-1* gene, the presence of additional polymorphism in genes positioned in the vicinity of the *NAM-1* gene may be used to determine whether a plant is progeny of a given parent plant. The presence of the same polymorphisms demonstrates that the plant is progeny of said parent plant.

The term "sequence identity" as used herein refers to the % of identical amino acids or nucleotides between a candidate sequence and a reference sequence following alignment. Thus, a candidate sequence sharing 80% amino acid identity with a reference sequence requires that, following alignment, 80% of the amino acids in the candidate sequence are identical to the corresponding amino acids in the reference sequence. Identity according to the present invention is determined by aid of computer analysis, such as, without limitations, the Clustal Omega computer alignment program for alignment of polypeptide sequences (Sievers et al. (2011 October 11) Molecular Systems Biology 7 :539, PMID: 21988835; Li et al. (2015 April 06) Nucleic Acids Research 43 (W1) :W580-4 PMID: 25845596; McWilliam et al., (2013 May 13) Nucleic Acids Research 41 (Web Server issue) :W597-600 PMID: 23671338), and the default parameters suggested therein. The Clustal Omega software is available from EMBL-EBI at https://www.ebi.ac.uk/Tools/msa/clustalo/. Using this program with its default settings, the mature (bioactive) part of a query and a reference polypeptide are aligned. The number of fully conserved residues are counted and divided by the length of the reference polypeptide. The MUSCLE or MAFFT algorithms may be used for alignment of nucleotide sequences. Sequence identities may be calculated in a similar way as indicated for amino acid sequences. Sequence identity as provided herein is thus calculated over the entire length of the reference sequence.

The term "stop codon" as used herein refers to a nucleotide triplet in the genetic code, which within mRNA results in termination of translation. The term "stop codon" as used herein also refers to a nucleotide triplet within a gene encoding the stop codon in mRNA. The stop codon in DNA typically has one of the following sequences: TAG, TAA or TGA.

The term "wild type NAM-1" as used herein refers to the wild type NAM-1 gene. As described by Wang et al. 2015, several haplotypes of NAM-1 exist, which can be considered wild type NAM-1. In particular, wild type NAM-1 as used herein may refer to a gene encoding a polypeptide of SEQ ID NO:1 or a polypeptide of SEQ ID NO:4. It is preferred that wild type NAM-1 is a gene encoding a polypeptide of SEQ ID NO:1.

By the term "wort" is meant a liquid extract of malt and/or cereal kernels, such as milled malt and/or milled cereal kernels and optionally additional adjuncts. Wort is in general obtained by mashing, optionally followed by "sparging", in a process of extracting residual sugars and other compounds from spent grains after mashing with hot water. Sparging is typically conducted in a lauter tun, a mash filter, or another apparatus to allow separation of the extracted water from spent grains. The wort obtained after mashing is generally referred to as "first wort", while the wort obtained after sparging is generally referred to as the "second wort". If not specified, the term wort may be first wort, second wort, or a combination of both. During conventional beer production, wort is boiled together with hops. Wort without hops, may also be referred to as "sweet wort", whereas wort boiled with hops may be referred to as "boiled wort" or simply as wort.

### NAM-1

The present invention provides a barley plant or a part thereof, wherein said barley plant carries a mutation in the *NAM-1* gene, wherein said mutated *NAM-1* gene encodes a mutant NAM-1 polypeptide, wherein the mutation is one of the following mutations
- a missense mutation in the NAC domain B of HvNAM-1;
- a missense mutation in the NAC domain A of HvNAM-1;
- a missense mutation resulting in a change from a charged amino acid to an uncharged amino acid in the NAC domain C of HvNAM-1; or
- a missense mutation resulting in a change from a Ser to Asn in the C-terminus of HvNAM-1.

The wild type gene encoding NAC transcription factor (NAM-1) may carry one or more different SNPs. For example, the wild type gene encoding NAM-1 may be of haplotype 2 or haplotype 7 according to Wang et al., 2015. Non-limiting examples of barley varieties of haplotype 7 includes cv. Optic and cv. Bowman. The complete coding sequence from barley *Hordeum vulgare* cultivar Optic is provided herein as SEQ ID NO:2. The skilled person will understand that other wild type barley plants comprises an *NAM-1* gene with a sequence differing from SEQ ID NO:2 to some extent. Preferably, a wild type barley *NAM-1* gene is a gene encoding a NAM-1 polypeptide of SEQ ID NO:1 or of SEQ ID NO:4. Very preferably, a barley wild type *NAM-1* gene is a gene encoding a NAM-1 polypeptide of SEQ ID NO:1.

According to Wang et al., 2015, barley plants of haplotype 2 encodes NAM-1 of SEQ ID NO:4, whereas barley plants of haplotype 7 encodes NAM-1 of SEQ ID NO:1.

### Barley plant carrying a mutation in the NAM-1 gene

The present disclosure provides a barley plant or a part thereof, wherein said barley plant carries a mutation in the *NAM-1* gene, wherein said mutated *NAM-1* gene encodes a mutant NAM-1 polypeptide. The mutation in the *NAM-1* gene may be any of the mutations described herein, however in preferred embodiments of the invention the mutation is a point mutation in the coding region of the *NAM-1* gene.

Moreover, the present disclosure provides a barley plant or a part thereof, wherein the grains of said barley plant have either a reduced GPC, or an increased GPC, wherein said barley plant carries a mutation in the *NAM-1* gene, wherein said mutated *NAM-1* gene encodes a mutant NAM-1 polypeptide.

The mutant NAM-1 polypeptide encoded by said mutant *NAM-1* gene may be any mutant NAM-1 polypeptide. A mutant NAM-1 polypeptide may be any polypeptide differing in sequence to wild type NAM-1. However preferably said mutant NAM-1 polypeptide contain one amino acid substitution. In particular, the mutant NAM-1 polypeptide may be any polypeptide differing in sequence to wild type NAM-1 of SEQ ID NO:1.

In particular, the mutant NAM-1 polypeptide may comprise a substitution of one amino acid in any one of the NAC domains A, B and C, or in the C-terminus of NAM-1.

In one embodiment of the disclosure the mutant NAM-1 polypeptide may comprise a missense mutation in the NAC domain B of HvNAM-1.

In one embodiment of the disclosure the mutant NAM-1 polypeptide may comprise a missense mutation in the NAC domain A of HvNAM-1.

In one embodiment of the disclosure the mutant NAM-1 polypeptide may comprise a missense mutation resulting in a change from a charged amino acid to an uncharged amino acid in the NAC domain C of HvNAM-1.

In one embodiment of the disclosure the mutant NAM-1 polypeptide may comprise a missense mutation resulting in a change from a Ser to Asn in the C-terminus of HvNAM-1.

### Barley plant carrying a mutation in the NAC domain B of NAM-1

In one embodiment of the disclosure the barley plant or part thereof carries a mutant NAM-1 polypeptide which may comprise a missense mutation in the NAC domain B of NAM-1 and has grains having a reduced grain protein content. Barley plants of haplotype 7 have been described as having lower GPC compared to barley plants of haplotype 2 (Wang et al., 2015). In one embodiment of the invention, the barley plant or part thereof carries a mutant NAM-1 polypeptide which may comprise a missense mutation in the NAC domain B of NAM-1 of SEQ ID NO:1 and has grains having a reduced grain protein content compared to a barley plant comprising a NAM-1 gene encoding NAM-1 of SEQ ID NO:1.

In one embodiment of the disclosure the mutant NAM-1 polypeptide may comprise a mutation of a conserved amino acid in the NAC domain B.

In one embodiment of the disclosure the mutant NAM-1 polypeptide may comprise a substitution of any amino acid in the NAC domain B to a sulphur-containing amino acid. In one embodiment of the disclosure the mutant NAM-1 polypeptide may comprise a substitution of an aliphatic amino acid to a sulphur-containing amino acid in the NAC domain B. Said sulphur containing amino acid may in particular be Met.

In one embodiment of the disclosure the mutant NAM-1 polypeptide may comprise a mutation in the NAC domain B, wherein said mutation is a substitution of a Val to a Met.

In one embodiment of the disclosure the mutated *NAM-1* gene encodes a mutant NAM-1, which is a polypeptide of SEQ ID NO:1 or SEQ ID NO:4 (preferably of SEQ ID NO:1) except that mutant NAM-1 polypeptide comprises a mutation in the NAC domain B of SEQ ID NO:1 or SEQ ID NO:4. Said mutation may for example be a mutation from any amino acid to a sulphur containing amino acid, e.g. to Met. Said NAC domain B may in particular correspond to or consist of amino acids 67 to 81 of SEQ ID NO:1 or SEQ ID NO:4.

In one embodiment of the disclosure the mutant NAM-1 polypeptide may comprise a mutation in a region corresponding to or consisting of the region spanning from amino acids 67 to 75 of SEQ ID NO:1 or SEQ ID NO:4. Said mutation may for example be a mutation from any amino acid to a sulphur containing amino acid, e.g. to Met.

In one embodiment of the disclosure the mutant NAM-1 polypeptide may comprise a substitution of one amino acid corresponding to the amino acid at position 70 of SEQ ID NO.1 or SEQ ID NO:4. Thus, the mutated *NAM-1* gene may encode a mutant NAM-1, which is a polypeptide of SEQ ID NO:1 or SEQ ID NO:4 (preferably of SEQ ID NO:1) except that said mutant NAM-1 comprises a substitution at position 70 of SEQ ID NO.1 or SEQ ID NO:4.

In one embodiment of the disclosure the mutant NAM-1 polypeptide may comprise a substitution of an aliphatic amino acid to a sulphur-containing amino acid at a position corresponding to position 70 of SEQ ID NO.1 or SEQ ID NO:4. Thus, the mutated *NAM-1* gene may encode a mutant NAM-1 polypeptide, which is a polypeptide of SEQ ID NO:1 or SEQ ID NO:4 (preferably of SEQ ID NO:1) except that said mutant NAM-1 comprises a substitution of Val to a sulphur-containing amino acid at position 70 of SEQ ID NO:1 or SEQ ID NO:4.ln one embodiment of the disclosure the mutant NAM-1 polypeptide may comprise a substitution of Val to Met at a position corresponding to position 70 of SEQ ID NO.1 or SEQ ID NO:4. In one embodiment of the disclosure the mutant NAM-1 polypeptide consists of SEQ ID NO:1 expect that Val at position 70 has been substituted to Met.

### Barley plant carrying a mutation in the NAC domain A or C, or in the C-terminus of NAM-1

In one embodiment of the disclosure the barley plant or part thereof carries a mutant NAM-1 polypeptide which may comprise a mutation in the NAC domain A or C of NAM-1, or in the C-terminus of NAM-1, and has grains having an increased soluble nitrogen content and an increased protein content.

In some embodiments, the mutant NAM-1 polypeptide may comprise a mutation in the NAC domain A of NAM-1. In particular, said mutation may be a substitution of an amino acid in the NAC domain A. The NAC domain A may in particular correspond to or consist of amino acids 35 to 56 of SEQ ID NO:1 or SEQ ID NO:4.

In one embodiment of the disclosure the mutant NAM-1 polypeptide may comprise a substitution of any amino acid to an Ile in the NAC domain A, wherein the NAC domain A corresponds or consists of to amino acids 35 to 56 of SEQ ID NO:1 or SEQ ID NO:4.

In one embodiment of the disclosure the mutant NAM-1 polypeptide may comprise a substitution of a Val to an Ile in the NAC domain A, wherein the NAC domain A is amino acids 35 to 56 of SEQ ID NO:1 or SEQ ID NO:4.

In one embodiment of the mutant NAM-1 polypeptide may comprise a substitution of one amino acid corresponding to the amino acid at position 49 of SEQ ID NO:1 or SEQ ID NO:4. For example, the mutant NAM-1 polypeptide may comprise a substitution of a Val to an Ile at a position corresponding to position 49 of SEQ ID NO:1 or SEQ ID NO:4. Thus, the mutant NAM-1 polypeptide may comprise a substitution of a Val to an Ile at position 49 of SEQ ID NO:1 or SEQ ID NO:4.

In one embodiment of the disclosure the mutated *NAM-1* gene may encode a mutant NAM-1 polypeptide, wherein the mutant NAM-1 polypeptide is a polypeptide of SEQ ID NO:1 or SEQ ID NO:4 except that mutant NAM-1 comprises a substitution at position 49 of SEQ ID NO:1 or SEQ ID NO:4. Said substitution may for example be substitution of a Val to an Ile.

In one embodiment of the disclosure the mutant NAM-1 polypeptide consists of SEQ ID NO:1 expect that Val at position 49 has been substituted to an Ile.

In some embodiments, the mutant NAM-1 polypeptide may comprise a mutation in the NAC domain C of NAM-1, for example mutant NAM-1 polypeptide may comprise a mutation from a charged amino acid to an uncharged amino acid in the NAC domain C. The NAC domain C may in particular correspond to or consist of amino acids 103 to 125 of SEQ ID NO:1 or SEQ ID NO:4.

In one embodiment of the disclosure, the mutant NAM-1 polypeptide may comprise a substitution of a charged amino acid to an uncharged amino acid at a position corresponding to position 122 of SEQ ID NO:1 or SEQ ID NO:4. Thus, the mutated *NAM-1* gene may encode a mutant NAM-1, which is a polypeptide of SEQ ID NO:1 or SEQ ID NO:4except that mutant NAM-1 may comprise a substitution of a charged amino acid to an uncharged amino acid at position 122 of SEQ ID NO:1 or SEQ ID NO:4.

In one embodiment of the disclosure, the mutant NAM-1 polypeptide may comprise a substitution of an Asp to an Asn at a position corresponding to position 122 of SEQ ID NO:1 or SEQ ID NO:4. Thus, the mutated *NAM-1* gene may encode a mutant NAM-1, which is a polypeptide of SEQ ID NO:1 or SEQ ID NO:4 except that mutant NAM-1 may comprise a substitution of an Asp to an Asn at position 122 of SEQ ID NO:1.

In one embodiment of the disclosure the mutant NAM-1 polypeptide consists of SEQ ID NO:1 expect that Asp at position 122 has been substituted to an Asn.

In one embodiment of the disclosure, the mutant NAM-1 polypeptide may comprise a mutation in the C-terminus.

In one embodiment of the disclosure, the mutant NAM-1 polypeptide may comprise a mutation from a Ser to Asn in the C-terminus. Thus, the mutated *NAM-1* gene may encode a mutant NAM-1, which is a polypeptide of SEQ ID NO:1 or SEQ ID NO:4 except that mutant NAM-1 may comprise a mutation from a Ser to Asn in the C-terminus. The C-terminus may in particular correspond to amino acids 205 to 356 of SEQ ID NO:1 or SEQ ID NO:4.

In one embodiment of the disclosure, the mutant NAM-1 polypeptide may comprise a mutation from a Ser to Asn at a position corresponding to position 330 of SEQ ID NO:1 or SEQ ID NO:4. Thus, the mutated *NAM-1* gene may encode a mutant NAM-1, which is a polypeptide of SEQ ID NO:1 except that mutant NAM-1 may comprise a mutation from a Ser to Asn at position 330 of SEQ ID NO:1 or SEQ ID NO:4.

In one embodiment of the disclosure the mutant NAM-1 polypeptide consists of SEQ ID NO:1 expect that Ser at position 330 has been substituted to an Asn.

In one embodiment of the invention it is preferred that said mutant NAM-1 polypeptide does not carry a mutation at a position corresponding to amino acid 357 of SEQ ID NO:1. In particular, it is preferred that said mutant NAM-1 is not a polypeptide of SEQ ID NO:1 wherein the amino acid at position 357 has been substituted to a Threonine.

### Barley plant

The barley plant according to the invention may be any plant of the species *Hordeum vulgare*, including any breeding line or cultivar or variety.

"Wild barley", *Hordeum vulgare* ssp. spontaneum, is considered the progenitor of today's cultivated forms of barley. Domesticated, but heterogeneous mixtures of barley are referred to as barley landraces. Today, most of the landraces have been displaced in advanced agricultures by pure line cultivars. Compared with landraces, modern barley cultivars have numerous improved properties (Nevo, 1992; Pelger et al., 1992).

Within the present invention, the term "barley plant" comprises any barley plant, such as barley landraces or modern barley cultivars. Thus, the invention relates to any barley plant comprising a mutation in the *NAM-1* gene.

However, preferred barley plants for use with the present invention are modern barley cultivars or pure lines. The barley cultivar to be used with the present invention may, for example, be selected from the group consisting of Paustian, Sebastian, Quench, Celeste, Lux, Prestige, Saloon, Neruda, Harrington, Klages, Manley, Schooner, Stirling, Clipper, Franklin, Alexis, Blenheim, Ariel, Lenka, Maresi, Steffi, Gimpel, Cheri, Krona, Camargue, Chariot, Derkado, Prisma, Union, Beka, Kym, Asahi 5, KOU A, Swan Hals, Kanto Nakate Gold, Hakata No. 2, Kirin - choku No. 1, Kanto late Variety Gold, Fuji Nijo, New Golden, Satukio Nijo, Seijo No. 17, Akagi Nijo, Azuma Golden, Amagi Nijpo, Nishino Gold, Misato golden, Haruna Nijo, Scarlett, Rosalina and Jersey preferably from the group consisting of Haruna Nijo, Sebastian, Quench, Celeste, Lux, Prestige, Saloon, Neruda and Power, preferably from the group consisting of Paustian, Harrington, Klages, Manley, Schooner, Stirling, Clipper, Franklin, Alexis, Blenheim, Ariel, Lenka, Maresi, Steffi, Gimpel, Cheri, Krona, Camargue, Chariot, Derkado, Prisma, Union, Beka, Kym, Asahi 5, KOU A, Swan Hals, Kanto Nakate Gold, Hakata No. 2, Kirin - choku No. 1, Kanto late Variety Gold, Fuji Nijo, New Golden, Satukio Nijo, Seijo No. 17, Akagi Nijo, Azuma Golden, Amagi Nijpo, Nishino Gold, Misato golden, Haruna Nijo, Scarlett and Jersey preferably from the group consisting of Paustian, Haruna Nijo, Sebastian, Tangent, Lux, Prestige, Saloon, Neruda, Power, Quench, NFC Tipple, Barke, Class, Vintage, Applaus, Bowie, Broadway, Champ, Chanson, Charles, Chimbon, Cosmopolitan, Crossway, Dragoon, Ellinor, Embrace, Etoile, Evergreen, Flair, Highway, KWS Beckie, KWS Cantton, KWS Coralie, KWS Fantex, KWS Irina, KWS Josie, KWS Kellie, LG Diablo, LG Figaro, LG Nabuco, LG Tomahawk, Laureate, Laurikka, Lauxana, Luther, Odyssey, Ovation, Prospect, RGT Elysium, RGT Observer, RGT Planet, Rotator, Sarbi, Scholar, Subway and Golden Promise.

The barley plant may be in any suitable form. For example, the barley plant according to the invention may be a viable barley plant, a dried plant, a homogenized plant, or a milled barley kernel. The plant may be a mature plant, an embryo, a kernel, a germinated kernel, a malted kernel (e.g. in the form of green malt or kiln dried malt), a milled malted kernel, a milled kernel or the like.

Parts of barley plants may be any suitable part of the plant, such as kernels, embryos, leaves, stems, roots, flowers, or fractions thereof. A fraction may, for example, be a section of a kernel, embryo, leaf, stem, root, or flower. Parts of barley plants may also be a fraction of a homogenate or a fraction of a milled barley plant or kernel.

In one embodiment of the invention, parts of barley plants may be cells of said barley plant, such as viable cells that may be propagated in vitro in tissue cultures. In other embodiments, however, the parts of barley plants may be viable cells that are not capable of maturing into an entire barley plant, i.e. cells that are not a reproductive material.

It is preferred that the barley plant is a barley plant, which has not exclusively been obtained by means of an essentially biological process or is progeny thereof.

For example, the barley plant may comprise a mutation in the *NAM-1* gene, wherein said mutation has been induced by chemical and/or physical agents, such as sodium azide.

Thus, the barley plant may have been prepared by a method involving a step of induced mutagenesis or said barley plant may be progeny of a plant prepared by a method involving a step of induced mutagenesis. Said induced mutagenesis may for example be treatment with a mutagenizing chemical, such as sodium azide.

### Characteristics of barley plants carrying a mutation in the NAM-1 gene

The invention provides barley plants carrying a mutation in the *NAM-1* gene. One major advantage of such barley plants is that the grains of said barley plants have an altered grain protein content (GPC). In fact, reduced GPC is often an advantageous trait for barley malt and beer production. The GPC in general correlates well with the malt protein content. Thus, malt prepared from barley grains with high GPC will in general also have a high content of protein, whereas malt prepared from barley grains with low GPC in general also have a low content of protein. Wort and other aqueous extracts of cereals and/or malt comprise proteins or degradation products thereof from said malt. Said proteins and nitrogen-containing degradation products thereof, e.g. peptides and amino acids are also referred to as "soluble nitrogen". The soluble nitrogen levels in a aqueous extract of a given cereal and/or malt from a given cereal also correlate to some extent with the GPC of said cereal. Thus aqueous extracts of cereals with high GPC and/or of malt from cereals with high GPC also in general has a high soluble protein level, whereas aqueous extracts of cereals with low GPC and/or of malt from cereals with low GPC also in general has a low soluble protein level.

Whereas a low GPC frequently is an advantageous trait for barley for beer production then a high GPC in grains is an advantageous trait for barley used in food production because of its positive effects on quality and nutritional value.

### Reduced GPC

Thus, in one embodiment the barley plants of the invention have grains with a low GPC. This is in particular the case for barley plants carrying a missense mutation in the NAC domain B of HvNAM-1, e.g. barley plants carrying a mutation of the amino acid corresponding to the amino acid at position 70 of SEQ ID NO.1 or SEQ ID NO:4. Interesting, the barley plants of the invention may have a GPC, which is lower compared to a barley plant comprising a NAM-1 gene encoding wild type NAM-1 of SEQ ID NO:1, which correspond to haplotype 7 (Wang et al., 2015).

In one embodiment the barley plants of the invention has a GPC, which is at least 3% lower, for example at least 5% lower, such as at least 7% lower, for example at least 9% lower than the GPC of a barley plant carrying a *NAM-1* gene encoding NAM-1 of SEQ ID NO:1, but otherwise of the same genotype, when cultivated under the same conditions.

In one embodiment the barley plants of the invention has a GPC, which is at least 3% , such as in the range of 3 to 15%, such as in the range of 3 to 12%, for example in the range of 3 to 10% lower than the GPC of a barley plant carrying a *NAM-1* gene encoding NAM-1 of SEQ ID NO:1, but otherwise of the same genotype, when cultivated under the same conditions.

The GPC in general correlates well with the malt protein content. Accordingly, in one embodiment malt prepared from the barley plants of the invention has a protein content, which is at least 3% , such as in the range of 3 to 15%, such as in the range of 3 to 12%, for example in the range of 3 to 10% lower than the protein content of malt prepared from a barley plant carrying a *NAM-1* gene encoding NAM-1 of SEQ ID NO:1, but otherwise of the same genotype, when cultivated under the same conditions.

In one embodiment, wort prepared from the barley plant of the invention has a soluble nitrogen content reduced by at least 3%, preferably reduced by at least 4% compared to wort prepared from a barley plant carrying a *NAM-1* gene encoding NAM-1 of SEQ ID NO:1, but otherwise of the same genotype, when said barley plants are cultivated under the same conditions and said worts are prepared in the same manner. Said wort is preferably prepared by mashing malt prepared from grains of aforementioned barley plants.

In one embodiment, wort prepared from the barley plant of the invention has a soluble nitrogen content reduced by in the range of 3 to 25%, such as in the range of 3 to 20%, for example in the range of 3 to 15%, such as in the range of 4 to 15% compared to wort prepared from a barley plant carrying a *NAM-1* gene encoding NAM-1 of SEQ ID NO:1, but otherwise of the same genotype, when said barley plants are cultivated under the same conditions and said worts are prepared in the same manner. Said wort is preferably prepared by mashing malt prepared from grains of aforementioned barley plants.

### Increased GPC

In one embodiment the barley plants of the invention have grains with a high GPC. This is in particular the case for barley plants carrying:
- a missense mutation in the NAC domain A of HvNAM-1;
- a missense mutation resulting in a change from a charged amino acid to an uncharged amino acid in the NAC domain C of HvNAM-1; or
- a missense mutation resulting in a change from a Ser to Asn in the C-terminus of HvNAM-1.

In one embodiment the barley plants of the invention has a GPC, which is increased at 4%, such as increased at least 5%, for example increased at least 10%, for example increased at least 20%, such as increased at least 30% compared to the GPC of a barley plant carrying a *NAM-1* gene encoding NAM-1 of SEQ ID NO:1, but otherwise of the same genotype, when cultivated under the same conditions.

In one embodiment, wort prepared from the barley plant of the invention have a soluble nitrogen content increased at least 0,7%, such as increased at least 1%, preferably increased at least 2%, for example increased at least 5%, such as increased at least 10%, for example increased at least 15% compared to wort prepared from a barley plant carrying a *NAM-1* gene encoding NAM-1 of SEQ ID NO:1, but otherwise of the same genotype, when said barley plants are cultivated under the same conditions and said wort is prepared in the same manner.. Said wort is preferably prepared by mashing malt prepared from grains of aforementioned barley plants.

### Plant products

The invention also provides plant products prepared from a barley plant carrying a mutation in the *NAM-1* gene, e.g. any of the barley plants described herein.

The plant product may be any product prepared from a barley plant, for example a food, a feed or a beverage. Thus the plant product may be any of the beverages described herein below in the section "Beverage and method of production thereof". The plant product may also be an aqueous extract of the barley plant and/or of malt of said barley plant, for example the plant product may be wort. Said aqueous extract may for example be prepared as described herein below in the section "Aqueous extract and methods of production thereof".

In one embodiment the plant product may be malt, e.g. any of the malts described herein below in the section "Malt and methods of production thereof" or a malt based product, such as malt based beverages. Although the primary use of malt is for beverage production, it can also be utilized in other industrial processes, for example as an enzyme source in the baking industry, or in the food industry as a flavouring and colouring agent, e.g. in the form of malt or malt flour or indirectly as a malt syrup, etc. Thus, the plant product according to the invention may be any of the aforementioned products.

In another aspect, the plant products according to the invention comprise, or even consist of syrup, such as a barley syrup, or a barley malt syrup. The plant product may also be an extract of barley or malt. Thus, the plant product may be wort.

### Malt and methods of production thereof

The invention also provides malt prepared from a barley plant carrying a mutation in the *NAM-1* gene, for example any of the barley plants described herein.

Malt may be prepared by malting, i.e. by germination of steeped barley kernels in a process taking place under controlled environmental conditions. The germination is frequently followed by a drying step. Said drying step may preferably be kiln drying of the germinated kernels at elevated temperatures.

Thus, a method of malting according to the present invention preferably comprises the steps of:
(a) providing kernels of a barley plant, notably a barley plant, carrying a mutation in the *NAM-1* gene;
(b) steeping said barley kernels;
(b) germinating said barley kernel; and
(c) optionally drying said germinated barley kernels, preferably by kiln drying.

Steeping may be performed by any conventional method known to the skilled person. One non-limiting example involves steeping at a temperature in the range of 10 to 25°C with alternating dry and wet conditions. During steeping, for example, the barley kernels may be incubated wet for in the range of 30 min to 3 h followed by incubation dry for in the range of 30 min to 3 h and optionally repeating said incubation scheme in the range of 2 to 5 times. The final water content after steeping may, for example, be in the range of 40 to 50%.

Germination of grains may be performed by any conventional method known to the skilled person. One non-limiting example involves germination at a temperature in the range of 10 to 25°C, optionally with changing temperature in the range of 1 to 4 days.

The kiln drying may be performed at conventional temperatures, such as at least 75°C, for example in the range of 80 to 90°C, such as in the range of 80 to 85°C. Thus, the malt may, for example be produced by any of the methods described by Hough et al. (1982). However, any other suitable method for producing malt may also be used with the present invention, such as methods for production of specialty malts, including, but not limited to, methods of roasting the malt.

Malt may be further processed, for example by milling. Thus, the plant product according to the invention may be any kind of malt, such as unprocessed malt or milled malt, such as flour. Milled malt and flour thereof comprise chemical components of the malt and dead cells that lack the capacity to re-germinate.

Preferably milling is performed in a dry state, i.e. the malt is milled while dry. Thus, it is preferred that malt is not milled under water.

An advantage of malt prepared from barley plants carrying a missense mutation in the NAC domain B of the HvNAM-1 is that said malt may have a low protein content. Malt prepared from grains with low protein content may be characterised by a high content of fermentable sugars.

### Aqueous extract and methods of production thereof

The invention provides barley based beverages as well as methods of preparing the same, wherein the barley is a barley plant carrying a mutation in the *NAM-1* gene.

Frequently, methods for preparing a beverage comprise a step of preparing an aqueous extract of kernels of the barley plants of the invention and/or of malts prepared from barley plants of the invention.

The aqueous extract may, in general, be prepared by incubating barley flour and/or malt flour in water or in an aqueous solution. In particular, the aqueous extract may be prepared by mashing.

In general said aqueous solution may be water, such as tap water to which one or more additional agents may be added. The additional agents may be present in the aqueous solution from the onset or they may be added during the process of preparing an aqueous extract. Said additional agents may be enzymes. Thus, the aqueous solution may comprise one or more enzymes. Said enzymes may be added to the aqueous solution from the onset, or subsequently, during the process.

Said enzymes may, for example, be one or more hydrolytic enzymes. Suitable enzymes include lipases, starch degrading enzymes (e.g. amylases), glucanases [preferably (1-4)- and/or (1,3;1,4)-β-glucanases], and/or xylanases (such as arabinoxylanases), and/or proteases, or enzyme mixtures comprising one or more of the aforementioned enzymes, e.g. Cereflo, Ultraflo, or Ondea Pro (Novozymes). For example,the aqueous solution may comprise one or more hydrolytic enzymes selected from the group consisting of α-amylase, β-amylase, limit dextrinase, pullulanase, β--glucanase (e.g. endo-(1,3;1,4)-β-glucanase or endo-1,4-β-glucanase),xylanase (e.g. endo- or exo-1,4-xylanase, an arabinofuranosidase or a ferulic acid esterase), glucoamylase and protease.

Said additional agents, preferably of food grade quality, may also be a salt, for example CaCl₂, or an acid, for example H₃PO₄.

The aqueous extract is generally prepared by incubation of the barley flour and/or malt flour in the aqueous solution at one or more predetermined temperature(s). Said predetermined temperature may also be referred to as "mashing temperature" herein. Said mashing temperatures may for example be conventional temperatures used for mashing. However, one advantage of the present invention is that the low gelatinisation temperature of the barley plants of the invention may allow use of relatively low mashing temperature. In particular, the mashing in temperature may be relatively low. The mashing temperature is in general either kept constant (isothermal mashing), or gradually increased, for example increased in a sequential manner. In either case, soluble substances in the barley grains and/or malt are liberated into said aqueous solution thereby forming an aqueous extract.

The mashing temperature(s) are typically temperature(s) in the range of 30 to 90°C, such as in the range of 40 to 85°C, for example in the range of 50 to 85°C. The mashing temperatures may be chosen according to the barley type used. In particular, a relatively low mashing-in temperature may be used, e.g. a temperature in the range of 50-60°C. In a preferred embodiment the mashing in temperature is higher than the gelatinisation temperature of the starch of the barley plant, for example in the range of 0.5 to 5°C higher than the gelatinisation temperature of the starch of the barley plant. Frequently, incubation with the aqueous solution includes a final step of heating to a higher temperature, e.g. to a temperature in the range of 75 to 80°C.

Subsequent to incubation in the aqueous solution in e.g. a mashing vessel, the aqueous solution may be transferred to another container, e.g. a lauter tun and incubated for additional time at elevated temperature.

Non-limiting examples of useful mashing protocols can be found in the literature of brewing, e.g. in Hough et al. (supra).

Mashing (i.e. incubation of the barley flour and/or malt flour in aqueous solution) can occur in the presence of adjuncts, which is understood to comprise any carbohydrate source other than malt, such as, but not limited to, barley, barley syrups, or maize, or rice - either as whole kernels or processed products like grits, syrups or starch. All of the aforementioned adjuncts may be used principally as an additional source of extract (syrups are typically dosed during wort heating). The requirements for processing of the adjunct in the brewery depend on the state and type of adjunct used, and in particular on the starch gelatinisation or liquefaction temperatures. If the gelatinisation temperature is above that for the barley plant starch of the invention, then starch of the adjunct may be gelatinized before addition to the mash.

After incubation in the aqueous solution, the aqueous extract may typically be separated, e.g. through filtration into the aqueous extract and residual non-dissolved solid particles, the latter also denoted "spent grain". Filtering may for example be performed in a lauter tun. Alternatively, the filtering may be filtering through a mash filter. The aqueous extract thus obtained may also be denoted "first wort". Additional liquid, such as water may be added to the spent grains during a process also denoted sparging. After sparging and filtration, a "second wort" may be obtained. Further worts may be prepared by repeating the procedure. Thus, the aqueous extract may be wort, e.g. a first wort, a second wort, a further wort or a combination thereof.

One advantage of aqueous extracts prepared from barley plants carrying a missense mutation in the NAC domain B of HvNAM-1 is that they typically have a low level of proteins and/or amino acids. Low levels of amino acids may be advantageous in order to avoid formation of Strecker aldehydes.

### Beverage and method of production thereof

The present invention also provides barley based beverages and methods of producing such beverages, wherein the barley is a barley plant carrying a mutation in the *NAM-1* gene.

Said beverage may be an alcoholic barley based beverages or non-alcoholic barley based beverages. Alcoholic barley based beverages may for example be beer or a distilled alcohol.

Said beer may be any kind of beer, for example lager or ale. Thus, the beer may for example be selected from the group consisting of altbier, Amber ale, Barley wine, Berliner Weisse, Bière de Garde, Bitter, Blonde Ale, Bock, Brown ale, California Common, Cream Ale, Dortmunder Export, Doppelbock, Dunkel, Dunkelweizen, Eisbock, Fruit Iambic, Golden Ale, Gose, Gueuze, Hefeweizen, Helles, India pale ale, Kölsch, Lambic, Light ale, Maibock, Malt liquor, Mild, Märzenbier, Old ale, Oud bruin, Pale ale, Pilsener, Porter, Red ale, Roggenbier, Saison, Scotch ale, Steam beer, Stout, Schwarzbier, lager, Witbier, Weissbier and Weizenbock.

Said distilled alcohol may be any kind of distilled alcohol. In particular the distilled alcohol may be based on a barley, e.g. a malted barley, e.g. a barley malt. Non-limiting examples of such distilled alcohol include whiskey and vodka.

The beverage may be a non-alcoholic beverage, such as a non-alcoholic barley based beverage, e.g. non-alcoholic beer or non-alcoholic malt beverages, such as maltina.

The beverage may for example be prepared by a method comprising the steps of:
(i) Providing kernels of a barley plant according to the invention and/or malt prepared from kernels of a barley plant according to the invention
(ii) Preparing an aqueous extract of said kernels and/or said malt, e.g. as described herein below in the section preparing aqueous extract
(iii) processing said aqueous extract into a beverage.

The aqueous extract may be boiled with or without hops where after it may be referred to as boiled wort. First, second and further worts may be combined, and thereafter subjected to boiling. The aqueous extract may be boiled for any suitable amount of time, e.g. in the range of 60 min to 120 min.

Step (iii) may in particular comprises fermentation of said aqueous extract, e.g. by fermentation of wort. Thus, the beverage may be prepared by fermentation of the aqueous extract with yeast.

Once the aqueous extract has been prepared it may be processed into beer by any method including conventional brewing methods. Non-limited descriptions of examples of suitable methods for brewing can be found, for example, in publications by Hough et al. (1982). Numerous, regularly updated methods for analyses of barley and beer products are available, for example, but not limited to, American Association of Cereal Chemists (1995), American Society of Brewing Chemists (1992), European Brewery Convention (1998), and Institute of Brewing (1997). It is recognized that many specific procedures are employed for a given brewery, with the most significant variations relating to local consumer preferences. Any such method of producing beer may be used with the present invention.

The first step of producing beer from the aqueous extract preferably involves boiling said aqueous extract as described herein above, followed by a subsequent phase of cooling and optionally whirlpool rest. One or more additional compounds may be added to the aqueous extract, e.g. one or more of the additional compounds described below in the section "Additional compounds". After being cooled, the aqueous extract may be transferred to fermentation tanks containing yeast, e.g. brewing yeast, such as *S*. *pastorianus* or *S*. *cerevisiae.* The aqueous extract may be fermented for any suitable time period, in general in the range of 1 to 20 days, such as 1 to 10 days. The fermentation is performed at any useful temperature e.g. at a temperature in the range of 10 to 20°C. The methods may also comprise addition of one or more enzymes, e.g. one or more enzymes may be added to the wort prior to or during fermentation. In particular, said enzyme may be a proline-specific endoprotease. A non-limiting examples of a proline-specific endoprotease is "Brewer's Clarex" available from DSM. In other embodiments, no exogenous enzymes are added during the methods.

During the several-day-long fermentation process, sugar is converted to alcohol and CO₂ concomitantly with the development of some flavour substances. The fermentation may be terminated at any desirable time, e.g. once no further drop in %P is observed.

Subsequently, the beer may be further processed, for example chilled. It may also be filtered and/or lagered - a process that develops a pleasant aroma and a less yeast-like flavour. Additives may also be added. Furthermore, CO₂ may be added. Finally, the beer may be pasteurized and/or filtered, before it is packaged (e.g. transferred to containers or kegs, bottled or canned). The beer may also be pasteurized by standard methods.

One advantage of beverages prepared from barley plants carrying a missense mutation in the NAC domain B of HvNAM-1 is that they typically have a low level of proteins and/or amino acids. Low levels of amino acids may be advantageous in order to avoid formation of Strecker aldehydes. In particular, the amino acids Met, Val, Ile, Leu and Phe are precursors of the Strecker aldehydes methional, 2-methylpropanal, 2-methylbutanal, 3-methylbutanal and phenylacetaldehyde, respectively. Thus, high levels of Met, Val, Ile, Leu and/or Phe are undesirable in beverages, such as beer, as they can result in formation of Strecker aldehyde off-flavours during storage.

Thus, it is preferred that the beverages prepared from the barley plants of the invention contains a level of amino acids, which is at least 10%, such as at least 20%, preferably at least 30% lower than the level of amino acids in a beverage prepared in the same manner from a barley plant carrying a *NAM-1* gene encoding NAM-1 of SEQ ID NO:1, but otherwise of the same genotype and cultivated under the same conditions.

It is preferred that the beverages prepared from the barley plants of the invention contains a level of Met, which is at least 10%, such as at least 15% lower, than the level of Met in a beverage prepared in the same manner from a barley plant carrying a *NAM-1* gene encoding NAM-1 of SEQ ID NO:1, but otherwise of the same genotype and cultivated under the same conditions.

It is preferred that the beverages prepared from the barley plants of the invention contains a level of Val, which is at least 20%, such as at least 30%, preferably at least 40% lower, than the level of Val in a beverage prepared in the same manner from a barley plant carrying a *NAM-1* gene encoding NAM-1 of SEQ ID NO:1, but otherwise of the same genotype and cultivated under the same conditions.

It is preferred that the beverages prepared from the barley plants of the invention contains a level of Ile, which is at least 20%, such as at least 30%, preferably at least 40% lower, than the level of Ile in a beverage prepared in the same manner from a barley plant carrying a *NAM-1* gene encoding NAM-1 of SEQ ID NO:1, but otherwise of the same genotype and cultivated under the same conditions.

It is preferred that the beverages prepared from the barley plants of the invention contains a level of Leu, which is at least 20%, such as at least 30%, preferably at least 40%, for example at least 50% lower, than the level of Leu in a beverage prepared in the same manner from a barley plant carrying a *NAM-1* gene encoding NAM-1 of SEQ ID NO:1, but otherwise of the same genotype and cultivated under the same conditions.

It is preferred that the beverages prepared from the barley plants of the invention contains a level of Phe, which is at least 20%, such as at least 30%, preferably at least 40% lower, than the level of Phe in a beverage prepared in the same manner from a barley plant carrying a *NAM-1* gene encoding NAM-1 of SEQ ID NO:1, but otherwise of the same genotype and cultivated under the same conditions.

### Additional compounds

The methods of the invention may comprise the step of adding one or more additional compounds. Said additional compounds may for example be a flavor compound, a preservative, a functional ingredient, a color, a sweetener, a pH regulating agent or a salt. The pH regulating agent may for example be a buffer or an acid, such as phosphoric acid.

Functional ingredients may be any ingredient added to obtain a given function. Preferably a functional ingredient renders the beverage healthier. Non-limiting examples of functional ingredients includes vitamins or minerals The preservative may be any food grade preservative, for example it may be benzoic acid, sorbic acid, sorbates (e.g. potassium sorbate), sulphites and/or salts thereof.

The additional compound may also be CO₂. In particular, CO₂ may be added to obtain a carbonated beverage.

The flavour compound to be used with the present invention may be any useful flavour compound. The flavour compound may for example be selected from the group consisting of aromas, plant extracts, plant concentrates, plant parts and herbal infusions. In particular the flavor compounds may be hops.

### Method of preparing a barley plant carrying a mutation in the NAM-1 gene

Barley plants carrying a mutation in the *NAM-1* gene may be prepared in any useful manner.

For example, such barley plants can be prepared by a method comprising the steps of:
- subjecting a plurality of barley plants or barley kernels to random mutagenesis, e.g. by irradiation or chemical treatment, e.g. treatment with sodium azide;
- identifying barley plants or barley kernels carrying a mutation in the *NAM-1* gene.

Such methods may also include one or more steps of reproducing said barley plants/barley kernels in order to obtain multiple barley plants/kernels each carrying random mutations.

In particular, barley plants carrying a particular mutation in *NAM-1* gene may be prepared and identified essentially as described in international patent application WO 2018/001884 using primers and probes designed to identify a mutation in the *NAM-1* gene. Examples of useful primers and probes are described herein below in Example 1.

Barley plants carrying a mutation in the *NAM-1* gene may also be prepared using various site directed mutatgenesis methods, which for example can be designed based on the sequence of the *NAM-1* gene, which is accessible under the GenBank accession number DQ869678.1 and provided herein as SEQ ID NO:3. In one embodiment, the barley plant is prepared using any one of CRISPR, a TALEN, a zinc finger, meganuclease, and a DNA-cutting antibiotic as described in WO 2017/138986. In one embodiment, the barley plant is prepared using CRISPR/cas9 technique, e.g. using RNA-guided Cas9 nuclease. This may be done as described in Lawrenson et al., Genome Biology (2015) 16:258; DOI 10.1186/s13059-015-0826-7 except that the single guide RNA sequence is designed based on the genes sequences provided herein. In one embodiment, the barley plant is prepared using a combination of both TALEN and CRISPR/cas9 techniques, e.g. using RNA-guided Cas9 nuclease. This may be done as described in Holme et al., Plant Mol Biol (2017) 95:111-121; DOI: 10.1007/s11103-017-0640-6) except that the TALEN and single guide RNA sequence are designed based on the genes sequences provided herein.

In one embodiment, the barley plant is prepared using homology directed repair, a combination of a DNA cutting nuclease and a donor DNA fragment. This may be done as described in Sun et al., Molecular Plant (2016) 9:628-631; DOI: https://doi.org/10.1016/j.molp.2016.01.001 except that the DNA cutting nuclease is designed based on the genes sequences provided herein and the donor DNA fragment is designed based on the coding sequence of the mutated barley variant provided herein.

In one embodiment of the invention, the objective is to provide agronomical useful barley plants carrying a mutation in the *NAM-1* gene. In addition to the mutation in the *NAM-1* gene, there are additional factors which also may be considered in the art of generating a commercial barley variety useful for malting and/or brewing and/or as base for beverages, for example kernel yield and size, and other parameters that relate to malting performance or brewing performance. Since many - if not all - relevant traits have been shown to be under genetic control, the present invention also provides modern, homozygous, high-yielding malting cultivars, which may be prepared from crosses with the barley plants that are disclosed in the present publication. The skilled barley breeder will be able to select and develop barley plants, which - following crossings with other barley plants - will result in superior cultivars. Alternatively, the breeder may utilize plants of the present invention for further mutagenesis to generate new cultivars carrying additional mutations in addition to the mutation of the *NAM-1* gene.

The invention also comprise barley plants carrying a mutation in the *NAM-1* gene prepared from plant breeding method, including methods of selfing, backcrossing, crossing to populations, and the like. Backcrossing methods can be used with the present invention to introduce into another cultivar the mutation of the *NAM-1* gene.

A way to accelerate the process of plant breeding comprises the initial multiplication of generated mutants by application of tissue culture and regeneration techniques. Thus, another aspect of the present invention is to provide cells, which upon growth and differentiation produce barley plants carrying the mutation of the *NAM-1* gene. For example, breeding may involve traditional crossings, preparing fertile anther-derived plants or using microspore culture.

### Items

The invention may further be defined by any of the following items:
1. A barley plant or a part thereof, wherein said barley plant carries a mutation in the *NAM-1* gene, wherein said mutated *NAM-1* gene encodes a mutant NAM-1 polypeptide, wherein the mutation is one of the following mutations
   - a missense mutation in the NAC domain B of HvNAM-1;
   - a missense mutation in the NAC domain A of HvNAM-1;
   - a missense mutation resulting in a change from a charged amino acid to an uncharged amino acid in the NAC domain C of HvNAM-1; or
   - a missense mutation resulting in a change from a Ser to Asn in the C-terminus of HvNAM-1.
2. The barley plant or a part thereof according to item 1, wherein said mutated *NAM-1* gene encodes a mutant NAM-1 polypeptide carrying a mutation of a conserved amino acid in the NAC domain B.
3. The barley plant or a part thereof according to any one of the preceding items, wherein said mutated *NAM-1* gene encodes a mutant NAM-1 polypeptide carrying a substitution of an aliphatic amino acid to a sulphur-containing amino acid in the NAC domain B.
4. The barley plant or a part thereof according to any one of the preceding items, wherein said mutated *NAM-1* gene encodes a mutant NAM-1 polypeptide carrying a mutation in the NAC domain B and wherein said mutation is a substitution of a Val to a Met.
5. The barley plant or a part thereof according to any one of the preceding items, wherein said mutated *NAM-1* gene encodes a mutant NAM-1 polypeptide of SEQ ID NO:1 except that mutant NAM-1 comprises a mutation in the NAC domain B.
6. The barley plant or a part thereof according to any one of the preceding items, wherein said mutated *NAM-1* gene encodes a mutant NAM-1 polypeptide of SEQ ID NO:4 except that mutant NAM-1 comprises a mutation in the NAC domain B.
7. The barley plant or a part thereof according to item 5, wherein the NAC domain B corresponds to amino acids 67 to 81 of SEQ ID NO:1 or SEQ ID NO:4.
8. The barley plant or a part thereof according to item 5 and 6, wherein the NAC domain B is amino acids 67 to 81 of SEQ ID NO:1 or SEQ ID NO:4.
9. The barley plant or a part thereof according to any one of the preceding items, wherein said mutated *NAM-1* gene encodes a mutant NAM-1 polypeptide comprising a mutation in a region corresponding to the region spanning from amino acids 67 to 75 of SEQ ID NO:1 or SEQ ID NO:4.
10. The barley plant or a part thereof according to any one of the preceding items, wherein said mutated *NAM-1* gene encodes a mutant NAM-1 polypeptide of SEQ ID NO:1 except that mutant NAM-1 comprises a mutation in the region spanning from amino acids 67 to 75 of SEQ ID NO:1.
11. The barley plant or a part thereof according to any one of the preceding items, wherein said mutated *NAM-1* gene encodes a mutant NAM-1 polypeptide of SEQ ID NO:4 except that mutant NAM-1 comprises a mutation in the region spanning from amino acids 67 to 75 of SEQ ID NO:1.
12. The barley plant or a part thereof according to any one of the preceding items, wherein said mutated *NAM-1* gene encodes a mutant NAM-1 polypeptide comprising a substitution of one amino acid corresponding to amino acid at position 70 of SEQ ID NO.1 or SEQ ID NO:4.
13. The barley plant or a part thereof according to any one of the preceding items, wherein said mutated *NAM-1* gene encodes a mutant NAM-1 polypeptide comprising a substitution of an aliphatic amino acid to a sulphur-containing amino acid at a position corresponding to position 70 of SEQ ID NO.1 or SEQ ID NO:4.
14. The barley plant or a part thereof according to any one of the preceding items, wherein said mutated *NAM-1* gene encodes a mutant NAM-1 polypeptide comprising a substitution of Val to Met at a position corresponding to position 70 of SEQ ID NO.1 or SEQ ID NO:4.
15. The barley plant or a part thereof according to any one of the preceding items, wherein said mutated *NAM-1* gene encodes a mutant NAM-1 polypeptide of SEQ ID NO:1 except that said mutant NAM-1 comprises a substitution at position 70 of SEQ ID NO.1.
16. The barley plant or a part thereof according to any one of the preceding items, wherein said mutated *NAM-1* gene encodes a mutant NAM-1 polypeptide of SEQ ID NO:4 except that said mutant NAM-1 comprises a substitution at position 70 of SEQ ID NO:4.
17. The barley plant or a part thereof according to any one of the preceding items, wherein said mutated *NAM-1* gene encodes a mutant NAM-1 polypeptide of SEQ ID NO:1 except that mutant NAM-1 comprises a substitution of an aliphatic amino acid to a sulphur-containing amino acid at position 70 of SEQ ID NO:1.
18. The barley plant or a part thereof according to any one of the preceding items, wherein said mutated *NAM-1* gene encodes a mutant NAM-1 polypeptide of SEQ ID NO:4 except that mutant NAM-1 comprises a substitution of an aliphatic amino acid to a sulphur-containing amino acid at position 70 of SEQ ID NO:4.
19. The barley plant or a part thereof according to any one of the preceding items, wherein said mutated *NAM-1* gene encodes a mutant NAM-1 polypeptide of SEQ ID NO:1 except that mutant NAM-1 comprises a substitution of Val to Met at position 70 of SEQ ID NO:1.
20. The barley plant or a part thereof according to any one of the preceding items, wherein said mutated *NAM-1* gene encodes a mutant NAM-1 polypeptide of SEQ ID NO:4 except that mutant NAM-1 comprises a substitution of an aliphatic amino acid to a sulphur-containing amino acid at position 70 of SEQ ID NO:4.
21. The barley plant or a part thereof according to any one of the preceding items, wherein wort prepared from malt from grains of said barley plant have a reduced soluble nitrogen content compared to wort prepared from malt from grains of a barley plant carrying a *NAM-1* gene encoding NAM-1 of SEQ ID NO:1, but otherwise of the same genotype, when said barley plants are cultivated under the same conditions and said worts are prepared in the same manner.
22. The barley plant or a part thereof according to any one of the preceding items, wherein grains of said barley plant have a reduced protein content compared to grains of a barley plant carrying a *NAM-1* gene encoding NAM-1 of SEQ ID NO:1, but otherwise of the same genotype, when cultivated under the same conditions.
23. The barley plant or a part thereof according to any one of the preceding items, wherein wort prepared from malt from grains of said barley plant have a soluble nitrogen content reduced by at least 3%, preferably reduced by at least 4%, for example reduced by in the range of 3 to 20% compared to wort prepared from malt from grains of a barley plant carrying a *NAM-1* gene encoding NAM-1 of SEQ ID NO:1, but otherwise of the same genotype, when said barley plants are cultivated under the same conditions and said worts are prepared in the same manner.
24. The barley plant or a part thereof according to any one of the preceding items, wherein grains of said barley plant have a protein content reduced by at least 3% compared to grains of a barley plant carrying a *NAM-1* gene encoding NAM-1 of SEQ ID NO:1, but otherwise of the same genotype, when cultivated under the same conditions.
25. The barley plant or a part thereof according to any one of the preceding items, wherein malt prepared from grains of said barley plant have a protein content reduced by at least 3% compared to malt prepared from grains of a barley plant carrying a *NAM-1* gene encoding NAM-1 of SEQ ID NO:1, but otherwise of the same genotype, when cultivated under the same conditions.
26. The barley plant or a part thereof according to item 1, wherein said mutated *NAM-1* gene encodes a mutant NAM-1 polypeptide carrying a substitution of an amino acid in the NAC domain A, wherein the NAC domain A corresponds to amino acids 35 to 56 of SEQ ID NO:1 or SEQ ID NO:4.
27. The barley plant or a part thereof according to item 1 and 26, wherein said mutated *NAM-1* gene encodes a mutant NAM-1 polypeptide carrying a substitution of an amino acid in the NAC domain A, wherein the NAC domain A is amino acids 35 to 56 of SEQ ID NO:1 or SEQ ID NO:4.
28. The barley plant or a part thereof according to any one of items 1 and 26 to 27, wherein said mutated *NAM-1* gene encodes a mutant NAM-1 polypeptide of SEQ ID NO:1 or SEQ ID NO:4, except that that mutant NAM-1 carries a substitution of an amino acid in the NAC domain A, wherein the NAC domain A corresponds to amino acids 35 to 56 of SEQ ID NO:1 or SEQ ID NO:4.
29. The barley plant or a part thereof according to any one of items 1 and 26 to 28, wherein said mutated *NAM-1* gene encodes a mutant NAM-1 polypeptide carrying a substitution of a Val to an Ile in the NAC domain A, wherein the NAC domain A corresponds to amino acids 35 to 56 of SEQ ID NO:1 or SEQ ID NO:4.
30. The barley plant or a part thereof according to any one of items 1 and 26 to 29, wherein said mutated *NAM-1* gene encodes a mutant NAM-1 polypeptide carrying a substitution of a Val to an Ile in the NAC domain A, wherein the NAC domain A is amino acids 35 to 56 of SEQ ID NO:1 or SEQ ID NO:4.
31. The barley plant or a part thereof according to any one of items 1 and 26 to 30, wherein said mutated *NAM-1* gene encodes a mutant NAM-1 polypeptide comprising a substitution of a Val to an Ile at a position corresponding to position 49 of SEQ ID NO:1 or SEQ ID NO:4.
32. The barley plant or a part thereof according to any one of items 1 and 26 to 31, wherein said mutated *NAM-1* gene encodes a mutant NAM-1 polypeptide comprising a substitution of one amino acid corresponding to the amino acid at position 49 of SEQ ID NO:1 or SEQ ID NO:4.
33. The barley plant or a part thereof according to any one of items 1 and 26 to 32, wherein said mutated *NAM-1* gene encodes a mutant NAM-1 polypeptide of SEQ ID NO:1 or SEQ ID NO:4 except that mutant NAM-1 comprises a substitution at position 49 of SEQ ID NO:1 or SEQ ID NO:4.
34. The barley plant or a part thereof according to any one of items 1 and 26 to 33, wherein said mutated *NAM-1* gene encodes a mutant NAM-1 polypeptide of SEQ ID NO:1 or SEQ ID NO:4 except that mutant NAM-1 comprises a substitution of a Val to an Ile at position 49 of SEQ ID NO:1 or SEQ ID NO:4.
35. The barley plant or a part thereof according to item 1, wherein said mutated *NAM-1* gene encodes a mutant NAM-1 polypeptide carrying a mutation from a charged amino acid to an uncharged amino acid in the NAC domain C.
36. The barley plant or a part thereof according to any one of items 1 and 35, wherein said mutated *NAM-1* gene encodes a mutant NAM-1 polypeptide comprising a substitution of an amino acid in the NAC domain C, wherein the NAC domain C corresponds to amino acids 103 to 125 of SEQ ID NO:1 or SEQ ID NO:4.
37. The barley plant or a part thereof according to any one of claims 1 and 35 to 36, wherein said mutated *NAM-1* gene encodes a mutant NAM-1 polypeptide polypeptide of SEQ ID NO:1 or SEQ ID NO:4 except that mutant NAM-1 comprises a substitution of an amino acid in the NAC domain C, wherein the NAC domain C corresponds to amino acids 103 to 125 of SEQ ID NO:1 or SEQ ID NO:4.
38. The barley plant or a part thereof according to any one of items 1 and 35 to 37, wherein said mutated *NAM-1* gene encodes a mutant NAM-1 polypeptide of SEQ ID NO:1 or SEQ ID NO:4 except that mutant NAM-1 comprises a substitution of an amino acid in the NAC domain C, wherein the NAC domain C is amino acids 103 to 125 of SEQ ID NO:1 or SEQ ID NO:4.
39. The barley plant or a part thereof according to any one of items 1 and 35 to 38, wherein said mutated *NAM-1* gene encodes a mutant NAM-1 polypeptide comprising a substitution of a charged amino acid to an uncharged amino acid in the NAC domain C, wherein the NAC domain C corresponds to amino acids 103 to 125 of SEQ ID NO:1 or SEQ ID NO:4.
40. The barley plant or a part thereof according to any one of items 1 and 35 to 39, wherein said mutated *NAM-1* gene encodes a mutant NAM-1 polypeptide of SEQ ID NO:1 or SEQ ID NO:4 except that mutant NAM-1 comprises a substitution of a charged amino acid to an uncharged amino acid in the NAC domain C, wherein the NAC domain C is amino acids 103 to 125 of SEQ ID NO:1 or SEQ ID NO:4.
41. The barley plant or a part thereof according to any one of items 1 and 35 to 40, wherein said mutated *NAM-1* gene encodes a mutant NAM-1 polypeptide comprising a substitution of a charged amino acid to an uncharged amino acid at a position corresponding to position 122 of SEQ ID NO:1 or SEQ ID NO:4.
42. The barley plant or a part thereof according to any one of items 1 and 35 to 41, wherein said mutated *NAM-1* gene encodes a mutant NAM-1 polypeptide of SEQ ID NO:1 or SEQ ID NO:4 except that mutant NAM-1 comprises a substitution of a charged amino acid to an uncharged amino acid at position 122 of SEQ ID NO:1 or SEQ ID NO:4.
43. The barley plant or a part thereof according to any one of items 1 and 35 to 42, wherein said mutated *NAM-1* gene encodes a mutant NAM-1 polypeptide comprising a substitution of an Asp to an Asn at a position corresponding to position 122 of SEQ ID NO:1.
44. The barley plant or a part thereof according to any one of items 1 and 35 to 43, wherein said mutated *NAM-1* gene encodes a mutant NAM-1 polypeptide of SEQ ID NO:1 or SEQ ID NO:4 except that mutant NAM-1 comprises a substitution of an Asp to an Asn at position 122 of SEQ ID NO:1 or SEQ ID NO:4.
45. The barley plant or a part thereof according to item 1, wherein said mutated *NAM-1* gene encodes a mutant NAM-1 polypeptide comprising a mutation from a Ser to Asn in the C-terminus.
46. The barley plant or a part thereof according to item 1 and 45, wherein said mutated *NAM-1* gene encodes a mutant NAM-1 polypeptide comprising a mutation from a Ser to Asn in the C-terminus, wherein the C-terminus corresponds to amino acids 205 to 356 of SEQ ID NO:1 or SEQ ID NO:4.
47. The barley plant or a part thereof according to item 1 and 45 to 46, wherein said mutated *NAM-1* gene encodes a mutant NAM-1 polypeptide of SEQ ID NO:1 or SEQ ID NO:4 except that mutant NAM-1 comprises a mutation from a Ser to Asn in the C-terminus, wherein the C-terminus is amino acids 205 to 356 of SEQ ID NO:1 or SEQ ID NO:4.
48. The barley plant or a part thereof according to item 1 and 45 to 47, wherein said mutated *NAM-1* gene encodes a mutant NAM-1 polypeptide comprising a mutation from a Ser to Asn at a position corresponding to position 330 of SEQ ID NO:1 or SEQ ID NO:4.
49. The barley plant or a part thereof according to item 1 and 45 to 48, wherein said mutated *NAM-1* gene encodes a mutant NAM-1 polypeptide of SEQ ID NO:1 or SEQ ID NO:4 except that mutant NAM-1 comprises a mutation from a Ser to Asn at position 330 of SEQ ID NO:1 or SEQ ID NO:4.
50. The barley plant or a part thereof according to any one of items 26 to 49, wherein wort prepared from malt from grains of said barley plant have an increased soluble nitrogen content compared to wort prepared from malt from grains of a barley plant carrying a wild type *NAM-1* gene, but otherwise of the same genotype, when said barley plants are cultivated under the same conditions and said worts are prepared in the same manner.
51. The barley plant or a part thereof according to any one of items 26 to 50, wherein the grains of said barley plant have an increased protein content compared to the grains of a barley plant carrying a wild type *NAM-1* gene, but otherwise of the same genotype, when cultivated under the same conditions.
52. The barley plant or a part thereof according to any one of items 26 to 51, wherein wort prepared from malt from grains of said barley plant have a soluble nitrogen content increased at least 0,7%, such as increased at least 1%, preferably increased at least 2%, for example increased at least 5%, such as increased at least 10%, for example increased at least 15% compared to wort prepared from malt from grains of a barley plant carrying a wild type *NAM-1* gene, but otherwise of the same genotype, when said barley plants are cultivated under the same conditions and said worts are prepared in the same manner.
53. The barley plant or a part thereof according to any one of items 26 to 52, wherein the grains of said barley plant have a protein content increased at least 4%, such as increased at least 5%, for example increased at least 10%, for example increased at least 20%, such as increased at least 30% compared to the grains of a barley plant carrying a wild type *NAM-1* gene, but otherwise of the same genotype, when cultivated under the same conditions.
54. The barley plant according to any one of the preceding items, with the proviso that the barley plant is a barley plant, which has not exclusively been obtained by means of an essentially biological process or is progeny thereof.
55. The barley plant according to any one of the preceding items, wherein said mutation has been induced by chemical and/or physical agents.
56. The barley plant according to any one of the preceding items, wherein said plant has been prepared by a method involving a step of induced mutagenesis or said plant is progeny of a plant prepared by a method involving a step of induced mutagenesis.
57. A plant product comprising the barley plant according to any one of the preceding items or a part thereof.
58. The plant product according item 57, wherein the plant product is green malt or dried malt prepared from kernels of said barley plant.
59. The plant product according item 57, wherein the plant product is wort prepared from kernels of said barley plant and/or from green malt or dried malt comprising processed kernel(s) of said barley plant.
60. The plant product according to item 57, wherein the plant product is a beverage prepared from said barley plant of parts thereof.
61. The beverage according to item 60, wherein said beverage is prepared from kernels of said barley plant and/or from malt comprising processed kernel(s) of said barley plant.
62. The beverage according to any one of items 60 to 61, wherein the beverage is beer.
63. A method of preparing green malt, said method comprising the steps of
   a. providing kernels of a barley plant according to any one of items 1 to 56;
   b. steeping said kernels;
   c. germinating the steeped kernels under predetermined conditions.
64. A method of preparing dried malt, said method comprising the steps of
   a. providing kernels of a barley plant according to any one of items 1 to 56;
   b. steeping said kernels;
   c. germinating the steeped kernels under predetermined conditions;
   d. drying said germinated kernels.
65. A method of producing a beverage, said method comprising the steps of:
   a. Providing grains of a barley plant according to any one of items 1 to 56, and/or malt according to item 58;
   b. Preparing an aqueous extract of said grains and/or said malt
   c. processing said aqueous extract into a beverage.
66. The method according to item 65, wherein step c. comprises a step of
   - heating said aqueous extract optionally in the presence of hops or hops extract.
67. The method according to any one of items 65 to 66, wherein step c. comprises a step of
   - fermenting said aqueous extract with a microorganism.
68. The method according to any one of items 65 to 67, wherein step c. comprises the steps of
   - heating said aqueous extract optionally in the presence of hops or hops extract;
   - cooling the aqueous extract;
   - fermenting said aqueous extract with a microorganism, thereby producing a fermented beverage.
69. The method according to any one of items 67 and 68, wherein the microorganism is yeast.
70. The method according to any one of items 65 to 69, wherein the beverage is beer.
71. A method of preparing a barley plant, the method comprising the steps of
   a. providing barley grains; and
   b. randomly mutagenising said barley grains, thereby introducing a mutation in the in the *NAM-1 gene,* wherein said mutated *NAM-1 gene* encodes a mutant NAM-1 polypeptide carrying one of the following mutations
      i. a mutation in the NAC domain B
      ii. a mutation in the NAC domain A
      iii. a mutation from a charged amino acid to an uncharged amino acid in the NAC domain C; or
      iv. a mutation from a Ser to Asn in the C-terminus
   c. selecting barley grains or progeny thereof carrying said mutated *NAM-1* gene.
72. The method according to item 71, wherein the barley plant or the mutation is as defined in any one of items 1 to 56.

### Sequences

SEQ ID NO: 1
   Amino acid sequence of *NAM-1* of *Hordeum vulgare cv. Optic* 
MGSPDSSSGSAQKPPRHQHQHQPPPPRRQGSAPELPPGFRFHPTDEELVVHYLKKKAAKAPLPVTIIAEV
DLYKFDPWELPEKATFGEHEWYFFSPRDRKYANGARPNRAATSGYWKATGTDKPILASATGCGREKVGVK
KALVFYRGKPPRGLKTNWIMHEYRLTGASAGSTTTSRPPPVTGGSRAPASLRLDDWVLCRIYKKTSKAAA
AVGDEQRSMECEDSVEDAVTAYPPYATAGMAGAGAHGSNYVQLLHHHDSHEDNFQLDGLLTEHDVGLSAG
AASLGHLAAAARATKQFLAPSSSTPFNWLEASTGGSILPQARNFPGFNRSRNVGSMSLSSTADDMAGAVD
VSDGGNAVNAMYLPVQDGTYHQHVILGAPLAPEAIAGAATSGFQHHVQISGVNWNP
SEQ ID NO:2
   Coding sequence of the *NAM-1* gene of *Hordeum vulgare cv Optic* (Genbank accession number DQ869678.1) 
ATGGGCAGCCCGGACTCATCCTCCGGCTCGGCGCAAAAACCACCGCGGCATCAGCATCAGCATCAGCCGC
CGCCTCCGCGGCGGCAGGGCTCGGCGCCGGAGCTCCCTCCCGGCTTCCGGTTCCACCCGACGGACGAGGA
GCTGGTCGTGCACTACCTCAAGAAGAAGGCCGCCAAGGCGCCGCTCCCCGTCACCATCATCGCCGAGGTG
GACCTCTACAAGTTCGACCCATGGGAGCTCCCCGAGAAGGCGACCTTCGGGGAGCACGAGTGGTACTTCT
TCAGCCCGCGCGACCGCAAGTACGCCAACGGCGCGCGGCCGAACCGGGCGGCGACGTCGGGCTACTGGAA
GGCCACCGGCACGGACAAGCCTATCCTGGCCTCGGCCACCGGGTGCGGCCGGGAGAAGGTCGGCGTCAAG
AAGGCGCTCGTCTTCTACCGCGGGAAGCCGCCCAGGGGCCTCAAGACCAACTGGATCATGCATGAGTACC
GCCTCACCGGAGCCTCTGCTGGCTCCACCACCACCAGCCGGCCGCCGCCGGTGACCGGCGGGAGCAGGGC
CCCGGCCTCTCTCAGGTTGGACGACTGGGTGCTGTGCCGCATCTACAAGAAGACCAGCAAGGCCGCGGCC
GCGGTCGGAGATGAGCAGAGGAGCATGGAGTGCGAGGACTCCGTGGAGGACGCGGTCACCGCGTACCCGC
CCTACGCCACGGCGGGCATGGCCGGCGCAGGTGCGCATGGCAGCAACTACGTTCAACTGCTCCATCATCA
CGACAGCCACGAGGACAACTTCCAGCTAGACGGCCTGCTCACAGAACACGACGTCGGCCTCTCGGCGGGC
GCCGCCTCGCTGGGCCACCTTGCCGCGGCGGCGAGGGCCACCAAACAGTTCCTCGCCCCGTCGTCCTCAA
CCCCGTTCAACTGGCTCGAGGCGTCAACCGGTGGCAGCATCCTCCCACAGGCAAGGAATTTCCCTGGGTT
TAACAGGAGCAGAAACGTCGGCAGTATGTCGCTGTCATCCACGGCCGACGACATGGCTGGCGCGGTGGAC
GTCAGCGACGGAGGCAATGCGGTGAACGCCATGTATCTCCCCGTGCAAGACGGGACCTACCATCAGCATG
 
TCATCCTCGGAGCTCCGCTGGCGCCAGAGGCCATCGCGGGCGCCGCCACCTCTGGTTTCCAGCATCACGT
CCAAATATCCGGCGTGAACTGGAATCCCTGA
SEQ ID NO:3
   Genomic sequence of the *NAM-1* gene of *Hordeum vulgare cv Optic* (Genbank accession number DQ869678.1) 
atgggcagcc cggactcatc ctccggctcg gcgcaaaaac caccgcggca tcagcatcag
catcagccgc cgcctccgcg gcggcagggc tcggcgccgg agctccctcc cggcttccgg
ttccacccga cggacgagga gctggtcgtg cactacctca agaagaaggc cgccaaggcg
ccgctccccg tcaccatcat cgccgaggtg gacctctaca agttcgaccc atggagctc
cccggtatgt actactagtt agtactatgt ctatccctat ctcgtcgatc gtgcttgctt
gctctatcaa gcgccgtaat ttcccggtgc aattaaataa tcgaatccgt ccacgcatcc
atccatcatg ctttttatta tactgtgcac aagtattttt atattcttcc agtaagtaca
gcgcatgtat gtgatcctgt cgtcgtgctt gttcatgcgc tcgggcggga tcatcatcca
tcagagaagg cgaccttcgg ggagcacgag tggtacttct tcagcccgcg cgaccgcaag
tacgccaacg gcgcgcggcc gaaccgggcg gcgacgtcgg gctactggaa ggccaccggc
acggacaagc ctatcctggc ctcggccacc gggtgcggcc gggagaaggt cggcgtcaag
aaggcgctcg tcttctaccg cgggaagccg cccaggggcc tcaagaccaa ctggatcatg
catgagtacc gcctcaccgg agcctctgct ggctccacca ccaccagccg gccgccgccg
gtgaccggcg ggagcagggc cccggcctct ctcagggtac gtacttacac gtgtccatcg
cacggtctat cagtatttat ttattaacta ctctcgagct taattatggt attgttgata
gttgatgaag ttaattattg tacgccgtct catcgatcag ttggacgact gggtgctgtg
ccgcatctac aagaagacca gcaaggccgc ggccgcggtc ggagatgagc agaggagcat
ggagtgcgag gactccgtgg aggacgcggt caccgcgtac ccgccctacg ccacggcggg
catggccggc gcaggtgcgc atggcagcaa ctacgttcaa ctgctccatc atcacgacag
ccacgaggac aacttccagc tagacggcct gctcacagaa cacgacgtcg gcctctcggc
gggcgccgcc tcgctgggcc accttgccgc ggcggcgagg gccaccaaac agttcctcgc
cccgtcgtcc tcaaccccgt tcaactggct cgaggcgtca accggtggca gcatcctccc
acaggcaagg aatttccctg ggtttaacag gagcagaaac gtcggcagta tgtcgctgtc
atccacggcc gacgacatgg ctggcgcggt ggacgtcagc gacggaggca atgcggtgaa
cgccatgtat ctccccgtgc aagacgggac ctaccatcag catgtcatcc tcggagctcc
gctggcgcca gaggccatcg cgggcgccgc cacctctggt ttccagcatc acgtccaaat
atccggcgtg aactggaatc cctga
SEQ ID NO: 4
   Amino acid sequence of *NAM-1* of *Hordeum vulgare* of haplotype 2

MGSPDSSSGSAQKPPRHQHQHQPPPPRRQGSAPELPPGFRFHPTDEELVVHYLKKKAAKAPLPVTIIAEV
DLYKFDPWELPEKATFGEHEWYFFSPRDRKYPNGARPNRAATSGYWKATGTDKPILASATGCGREKVGVK
KALVFYRGKPPRGLKTNWIMHEYRLTGASAGSTTTSRPPPVTGGSRAPASLRLDDWVLCRIYKKTSKAAA
AVGDEQRSMECEDSVEDAVTAYPPYATAGMAGAGAHGSNYVQLLHHHDSHEDNFQLDGLLTEHDVGLSAG
AASLGHLAAAARATKQFLAPSSSTPFNWLEASTGGSILPQARNFPGFNRSRNVGSMSLSSTADDMAGAVD
VSDGGNAVNAMYLPVQDGTYHQHVILGAPLAPEAIAGAATSGFQHHVQISGVNWNP

### Examples

### Example 1. Screening for barley mutants with specific mutations in the gene for NAC transcription factor NAM-1

Altogether 5 mutants with a specific mutation, leading to the substitution of an amino acid residue in NAC transcription factor NAM-1 (HvNAM-1), were identified (Table 1).

**Table 1. Identified mutants.**

| Mutant (Internal ID) | Nucleotide change in coding sequence (SEQ ID NO:2 ) | Amino acid change in protein (SEQ ID NO:1) |
|---|---|---|
| Mutant 1 | G→A (989) | Ser→Asn (330) |
| Mutant 2 | C→T (1091) | Pro→Leu (364) |
| Mutant 3 | G→A (208) | Val→Met (70) |
| Mutant 4 | G→A (145) | Val→Ile (49) |
| Mutant 5 | G→A (364) | Asp→Asn (122) |

Mutant 3, mutant 4, and mutant 5 were identified using a ddPCR screening method essentially as described in international patent application WO 2018/001884. More specifically, a pool of randomly mutagenized barley grains (parent variety Paustian) was prepared, followed by preparation of an ordered library as described in international patent application WO 2018/001884 in WS1 and WS2 on p. 66-69 as well as in Examples 1 to 2. Paustian contains a wild type NAM-1 gene encoding NAM-1 of SEQ ID NO:1 (haplotype 7). The mutant 3, mutant 4 and mutant 5 were identified and selected as described in international patent application WO 2018/001884 in WS3 and WS4 on p. 67-72 as well as in Examples 3 to 15 using the primers and probes specified in Table 2 below.

Primers and probes were designed specifically for the identification of specific mutants at the NAM-1-locus, are described in Table 2.

**Table 2. Primers and probes designed for the specific mutants.**

| Mutant | Target-specific forward primer | Target-specific reverse primer | Mutant-specific detection probe labelled with 6-carboxyfluorescein (FAM) | Reference-specific detection probe labelled with *hexachlorofluorescein* (HEX) |
|---|---|---|---|---|
| Mutant 3 | CCGCTCCCCGTC A | AACTAGTAGTACA TACCGGG | CGCCGAGATGGA CCT | TCGCCGAGGTGGA C |
| Mutant 4 | CTTCCGGTTCCA CCC | GGCGGCCTTCTTC TT | CGAGGAGCTGAT CGTG | CGAGGAGCTGGTC GT |
| Mutant 5 | GTCGGGCTACTG GAAG | ACCCGGTGGCCG | ACCGGCACGAAC AAG | ACCGGCACGGACA A |

Mutant 1 and mutant 2 were identified using a direct sequencing approach of 6000 mutagenized M3 barley mutants (parent variety Quench) using specific primers (forward primer 5'- GCCGTCTCATCGATCAGTTG-3'; reverse primer 5'-TGGTGTCATTCGTTCAGGGA-3') to amplify parts of the HvNAM-1 locus.

### Example 2. Nitrogen content in malt generated from barley plants carrying a mutated NAM-1

Barley kernels of mutants 1-5 and of the reference (cv. Paustian and cv. Quench) were processed in triplicates, 50 g each sample). Dry barley kernels placed in stainless steel beakers were micromalted according to MEBAK approved method MEBAK R-110.00.008 of March 2016, except that a few parameters were adapted to handle smaller sample volumes. The micromalting involved steeping of barley kernels, germination and kiln drying.

Prior to measurements of protein and nitrogen content the malt samples were milled using a standard Foss Cyclotech mill equipped with a tungsten carbide grinding ring (Foss 10004463), nickel plated impeller (Foss 1000 2666) and a 1 mm outlet screen (Foss 10001989).

Total protein in malt was determined by the DUMAS combustion method using MEBAK approved method MEBAK R-200.20.042 of March 2016 referring to the method of determining total protein in barley MEBAK R-110.41.042 of March 2016. This method is comparable with Analytica-EBC 4.3.2, which refers to Analytica-EBC 3.3.2.
The result is shown in Figure 1A and in Table 3 below.

Soluble nitrogen was determined by the DUMAS combustion method using MEBAK approved method MEBAK R-205.11.042 of March 2016. This method is comparable with Analytica-EBC 4.9.3.
The result is shown in Figure 1B and table 3 below.

**Table 3. Content of protein and soluble nitrogen in malt produced from grains of mutant (1-5) barley plants.**

| Mutant | Grain Protein Content [% dm] | Reduction of GPC compared to WT (%) | Soluble nitrogen [mg/100 g dm] | Reduction of soluble nitrogen compared to WT (%) |
|---|---|---|---|---|
| Mutant 1 | 9,1 | 8.3 | 686 | -0.7 |
| Mutant 2 | 8,1 | -3.6 | 695 | 0.6 |
| Mutant 3 | 7,7 | -9.5 | 645 | -4.5 |
| Mutant 4 | 11,7 | 36.0 | 819 | 21.3 |
| Mutant 5 | 8,9 | 3.5 | 705 | 4.4 |
| Paustian (WT) | 8,6 | - | 675 | - |
| Quench (WT) | 8,4 | - | 691 | - |

Quench was the control for mutants 1 and 2; Paustian was the control for mutants 3, 4 and 5.

### Example 3

Mutant 3 and Paustian barley plants were grown in neighbouring plots in New Zealand in the season 17/18. The harvested grains were analysed as follows.

### Malting

Grains were malted according to standard procedures. Briefly, the malting time was in total 5 days. The steeping was performed at 18°C and concluded when 43% moisture content was reached. The germination was then performed at 14.5 °C. At the end of germination the green malt was kiln dried.

### Mashing and fermentation

The malt thus obtained was mashed. For mashing 100g malt flour and 300g water was mixed and incubated at 52°C for 30 min. Temperature was then increased to 65°C by ramping 1°C/min. The mash was incubated 50 minutes at 65°C before ramping to 78°C 1°C/min. Finally the mash was incubated at 78°C for 10 min. Water was then added to a total of 500g for the mash. After mixing 200ml of wort was taken out and boiled for 15 minutes and cooled to room temperature. Six g of yeast was then added to the wort and fermention was performed at room temperature for 7 days in order to obtain beer. The amino acid content of wort and beer was determined as described in Example 9 of European patent application EP3237601 using a UPLC with Photo Diode Array detector using the AccQ-Tag Ultra derivatization kit from Waters, essentially as described by the supplier.

The results are shown in Table 4 below.

**Table 4**

| **Barley line** | **Paustian** | **Mutant 3** | **Reduction compared to Paustian %** |
|---|---|---|---|
| Malt protein (% of dry matter) | 10,4 | 10,0 | -3,8% |
| Wort Soluble Nitrogen (mg/100 g dry matter) | 676 | 583 | -14% |
| Wort FAN (mg/100 g dry matter) | 117 | 94 | -20% |
| Wort total amino acids (mg/L) | 3762 | 2714 | -28% |
| Beer total amino acids (mg/L) | 2490 | 1577 | -37% |
| Beer Met (mg/l) | 21 | 17 | -19% |
| Beer Val (mg/l) | 204 | 107 | -48% |
| Beer Ile (mg/l) | 95 | 48 | -49% |
| Beer Leu (mg/l) | 184 | 79 | -57% |
| Beer Phe (mg/l) | 180 | 93 | -48% |

### References

H. Arzani, M. Basiri, F. Khatibi and G. Ghorbani, 2006, Nutritive value of some Zagros Mountain rangeland species, Small Ruminant Research, 65:128-135
C. Jamar, F. Loffet, P. Frettinger, L. Ramsay, M Fauconnier and P du Jardin, 2010, NAM-1 gene polymorphism and grain protein content in Hordeum, Journal of Plant Physiology 167: 497-501
Y. Wang, X. Ren, D. Sun and G. Sun, 2015, Origin of worldwide cultivated barley revealed by NAM-1 gene and grain protein content, Front. Plant. Sci. 6:803

## Claims

1. A barley plant or a part thereof, wherein said barley plant carries a mutation in the *NAM-1* gene, wherein said mutated *NAM-1* gene encodes a mutant NAM-1 polypeptide, wherein the mutation is one of the following mutations
• a missense mutation in the NAC domain B of HvNAM-1;
• a missense mutation in the NAC domain A of HvNAM-1;
• a missense mutation resulting in a change from a charged amino acid to an uncharged amino acid in the NAC domain C of HvNAM-1; or
• a missense mutation resulting in a change from a Ser to Asn in the C-terminus of HvNAM-1.

2. The barley plant or a part thereof according to any one of the preceding claims,
wherein said mutated *NAM-1* gene encodes a mutant NAM-1 polypeptide carrying a substitution of an aliphatic amino acid to a sulphur-containing amino acid in the NAC domain B.

3. The barley plant or a part thereof according to any one of the preceding claims,
wherein said mutated *NAM-1* gene encodes a mutant NAM-1 polypeptide of SEQ ID NO:1 except that mutant NAM-1 comprises a mutation in the NAC domain B or said mutated *NAM-1* gene encodes a mutant NAM-1 polypeptide of SEQ ID NO:4 except that mutant NAM-1 comprises a mutation in the NAC domain B, wherein the NAC domain B corresponds to amino acids 67 to 81 of SEQ ID NO:1 or SEQ ID NO:4.

4. The barley plant or a part thereof according to any one of the preceding claims,
wherein said mutated *NAM-1* gene encodes a mutant NAM-1 polypeptide of SEQ ID NO:1 or SEQ ID NO:4 except that said mutant NAM-1 comprises a substitution at position 70 of SEQ ID NO.1 or SEQ ID NO:4.

5. The barley plant or a part thereof according to any one of the preceding claims,
wherein said mutated *NAM-1* gene encodes a mutant NAM-1 polypeptide of SEQ ID NO:1 or SEQ ID NO:4 except that mutant NAM-1 comprises a substitution of Val to Met at position 70 of SEQ ID NO:1 or SEQ ID NO:4.

6. The barley plant or a part thereof according to any one of the preceding claims,
wherein grains of said barley plant have a protein content reduced by at least 3% compared to grains of a barley plant carrying a *NAM-1* gene encoding NAM-1 of SEQ ID NO:1, but otherwise of the same genotype, when cultivated under the same conditions.

7. The barley plant or a part thereof according to any one of the preceding claims,
wherein malt prepared from grains of said barley plant have a protein content reduced by at least 3% compared to malt prepared from grains of a barley plant carrying a *NAM-1* gene encoding NAM-1 of SEQ ID NO:1, but otherwise of the same genotype, when cultivated under the same conditions.

8. The barley plant or a part thereof according to claim 1, wherein said mutated *NAM-1* gene encodes a mutant NAM-1 polypeptide of SEQ ID NO:1 or SEQ ID NO:4, except that mutant NAM-1 comprises a substitution of an amino acid in the NAC domain A, wherein the NAC domain A corresponds to amino acids 35 to 56 of SEQ ID NO:1 or SEQ ID NO:4.

9. The barley plant or a part thereof according to claim 1, wherein said mutated *NAM-1* gene encodes a mutant NAM-1 polypeptide polypeptide of SEQ ID NO:1 or SEQ ID NO:4 except that mutant NAM-1 comprises a substitution of an amino acid in the NAC domain C, wherein the NAC domain C corresponds to amino acids 103 to 125 of SEQ ID NO:1 or SEQ ID NO:4.

10. The barley plant or a part thereof according to claim 1, wherein said mutated *NAM-1* gene encodes a mutant NAM-1 polypeptide of SEQ ID NO:1 or SEQ ID NO:4 except that mutant NAM-1 comprises a mutation from a Ser to Asn in the C-terminus, wherein the C-terminus is amino acids 205 to 356 of SEQ ID NO:1 or SEQ ID NO:4.

11. A plant product comprising the barley plant according to any one of the preceding claims or a part thereof.

12. The plant product according claim 15, wherein the plant product is selected from the group consisting of
• green malt prepared from kernels of said barley plant;
• dried malt prepared from kernels of said barley plant;
• wort prepared from kernels of said barley plant and/or from green malt or dried malt comprising processed kernel(s) of said barley plant; and
• a beverage, for example beer, prepared from said barley plant of parts thereof.

13. A method of preparing malt, said method comprising the steps of
a. providing kernels of a barley plant according to any one of claims 1 to 14;
b. steeping said kernels;
c. germinating the steeped kernels under predetermined conditions
d. optionally drying said germinated kernels.

14. A method of producing a beverage, said method comprising the steps of:
a. Providing grains of a barley plant according to any one of claims 1 to 14 and/or malt according to claim 16;
b. Preparing an aqueous extract of said grains and/or said malt
c. processing said aqueous extract into a beverage.

15. A method of preparing a barley plant, the method comprising the steps of
a. providing barley grains; and
b. randomly mutagenising said barley grains, thereby introducing a mutation in the in the *NAM-1 gene,* wherein said mutated *NAM-1 gene* encodes a mutant NAM-1 polypeptide carrying one of the following mutations
v. a mutation in the NAC domain B
vi. a mutation in the NAC domain A
vii. a mutation from a charged amino acid to an uncharged amino acid in the NAC domain C; or
viii. a mutation from a Ser to Asn in the C-terminus
c. selecting barley grains or progeny thereof carrying said mutated *NAM-1* gene.


SEQUENCE LISTING

<110>  Carlsberg A/S

<120>  Plant with low nitrogen levels

<130>  P4541EP00

<160>  4

<170>  PatentIn version 3.5

<210>  1
<211>  406
<212>  PRT
<213>  Hordeum vulgare cv. Optic

<400>  1

Met Gly Ser Pro Asp Ser Ser Ser Gly Ser Ala Gln Lys Pro Pro Arg
1               5                   10                  15

His Gln His Gln His Gln Pro Pro Pro Pro Arg Arg Gln Gly Ser Ala
            20                  25                  30

Pro Glu Leu Pro Pro Gly Phe Arg Phe His Pro Thr Asp Glu Glu Leu
        35                  40                  45

Val Val His Tyr Leu Lys Lys Lys Ala Ala Lys Ala Pro Leu Pro Val
    50                  55                  60

Thr Ile Ile Ala Glu Val Asp Leu Tyr Lys Phe Asp Pro Trp Glu Leu
65                  70                  75                  80

Pro Glu Lys Ala Thr Phe Gly Glu His Glu Trp Tyr Phe Phe Ser Pro
                85                  90                  95

Arg Asp Arg Lys Tyr Ala Asn Gly Ala Arg Pro Asn Arg Ala Ala Thr
            100                 105                 110

Ser Gly Tyr Trp Lys Ala Thr Gly Thr Asp Lys Pro Ile Leu Ala Ser
        115                 120                 125

Ala Thr Gly Cys Gly Arg Glu Lys Val Gly Val Lys Lys Ala Leu Val
    130                 135                 140

Phe Tyr Arg Gly Lys Pro Pro Arg Gly Leu Lys Thr Asn Trp Ile Met
145                 150                 155                 160

His Glu Tyr Arg Leu Thr Gly Ala Ser Ala Gly Ser Thr Thr Thr Ser
                165                 170                 175

Arg Pro Pro Pro Val Thr Gly Gly Ser Arg Ala Pro Ala Ser Leu Arg
            180                 185                 190

Leu Asp Asp Trp Val Leu Cys Arg Ile Tyr Lys Lys Thr Ser Lys Ala
        195                 200                 205

Ala Ala Ala Val Gly Asp Glu Gln Arg Ser Met Glu Cys Glu Asp Ser
    210                 215                 220

Val Glu Asp Ala Val Thr Ala Tyr Pro Pro Tyr Ala Thr Ala Gly Met
225                 230                 235                 240

Ala Gly Ala Gly Ala His Gly Ser Asn Tyr Val Gln Leu Leu His His
                245                 250                 255

His Asp Ser His Glu Asp Asn Phe Gln Leu Asp Gly Leu Leu Thr Glu
            260                 265                 270

His Asp Val Gly Leu Ser Ala Gly Ala Ala Ser Leu Gly His Leu Ala
        275                 280                 285

Ala Ala Ala Arg Ala Thr Lys Gln Phe Leu Ala Pro Ser Ser Ser Thr
    290                 295                 300

Pro Phe Asn Trp Leu Glu Ala Ser Thr Gly Gly Ser Ile Leu Pro Gln
305                 310                 315                 320

Ala Arg Asn Phe Pro Gly Phe Asn Arg Ser Arg Asn Val Gly Ser Met
                325                 330                 335

Ser Leu Ser Ser Thr Ala Asp Asp Met Ala Gly Ala Val Asp Val Ser
            340                 345                 350

Asp Gly Gly Asn Ala Val Asn Ala Met Tyr Leu Pro Val Gln Asp Gly
        355                 360                 365

Thr Tyr His Gln His Val Ile Leu Gly Ala Pro Leu Ala Pro Glu Ala
    370                 375                 380

Ile Ala Gly Ala Ala Thr Ser Gly Phe Gln His His Val Gln Ile Ser
385                 390                 395                 400

Gly Val Asn Trp Asn Pro
                405

<210>  2
<211>  1221
<212>  DNA

<213>  Hordeum vulgare cv Optic

<400>  2
atgggcagcc cggactcatc ctccggctcg gcgcaaaaac caccgcggca tcagcatcag      60

catcagccgc cgcctccgcg gcggcagggc tcggcgccgg agctccctcc cggcttccgg     120

ttccacccga cggacgagga gctggtcgtg cactacctca agaagaaggc cgccaaggcg     180

ccgctccccg tcaccatcat cgccgaggtg gacctctaca agttcgaccc atgggagctc     240

cccgagaagg cgaccttcgg ggagcacgag tggtacttct tcagcccgcg cgaccgcaag     300

tacgccaacg gcgcgcggcc gaaccgggcg gcgacgtcgg gctactggaa ggccaccggc     360

acggacaagc ctatcctggc ctcggccacc gggtgcggcc gggagaaggt cggcgtcaag     420

aaggcgctcg tcttctaccg cgggaagccg cccaggggcc tcaagaccaa ctggatcatg     480

catgagtacc gcctcaccgg agcctctgct ggctccacca ccaccagccg gccgccgccg     540

gtgaccggcg ggagcagggc cccggcctct ctcaggttgg acgactgggt gctgtgccgc     600

atctacaaga agaccagcaa ggccgcggcc gcggtcggag atgagcagag gagcatggag     660

tgcgaggact ccgtggagga cgcggtcacc gcgtacccgc cctacgccac ggcgggcatg     720

gccggcgcag gtgcgcatgg cagcaactac gttcaactgc tccatcatca cgacagccac     780

gaggacaact tccagctaga cggcctgctc acagaacacg acgtcggcct ctcggcgggc     840

gccgcctcgc tgggccacct tgccgcggcg gcgagggcca ccaaacagtt cctcgccccg     900

tcgtcctcaa ccccgttcaa ctggctcgag gcgtcaaccg gtggcagcat cctcccacag     960

gcaaggaatt tccctgggtt taacaggagc agaaacgtcg gcagtatgtc gctgtcatcc    1020

acggccgacg acatggctgg cgcggtggac gtcagcgacg gaggcaatgc ggtgaacgcc    1080

atgtatctcc ccgtgcaaga cgggacctac catcagcatg tcatcctcgg agctccgctg    1140

gcgccagagg ccatcgcggg cgccgccacc tctggtttcc agcatcacgt ccaaatatcc    1200

ggcgtgaact ggaatccctg a                                              1221

<210>  3
<211>  1585
<212>  DNA
<213>  Hordeum vulgare cv Optic

<400>  3
atgggcagcc cggactcatc ctccggctcg gcgcaaaaac caccgcggca tcagcatcag      60

catcagccgc cgcctccgcg gcggcagggc tcggcgccgg agctccctcc cggcttccgg     120

ttccacccga cggacgagga gctggtcgtg cactacctca agaagaaggc cgccaaggcg     180

ccgctccccg tcaccatcat cgccgaggtg gacctctaca agttcgaccc atgggagctc     240

cccggtatgt actactagtt agtactatgt ctatccctat ctcgtcgatc gtgcttgctt     300

gctctatcaa gcgccgtaat ttcccggtgc aattaaataa tcgaatccgt ccacgcatcc     360

atccatcatg ctttttatta tactgtgcac aagtattttt atattcttcc agtaagtaca 420

gcgcatgtat gtgatcctgt cgtcgtgctt gttcatgcgc tcgggcggga tcatcatcca 480

tcagagaagg cgaccttcgg ggagcacgag tggtacttct tcagcccgcg cgaccgcaag 540

tacgccaacg gcgcgcggcc gaaccgggcg gcgacgtcgg gctactggaa ggccaccggc 600

acggacaagc ctatcctggc ctcggccacc gggtgcggcc gggagaaggt cggcgtcaag 660

aaggcgctcg tcttctaccg cgggaagccg cccaggggcc tcaagaccaa ctggatcatg 720

catgagtacc gcctcaccgg agcctctgct ggctccacca ccaccagccg gccgccgccg 780

gtgaccggcg ggagcagggc cccggcctct ctcagggtac gtacttacac gtgtccatcg 840

cacggtctat cagtatttat ttattaacta ctctcgagct taattatggt attgttgata 900

gttgatgaag ttaattattg tacgccgtct catcgatcag ttggacgact gggtgctgtg 960

ccgcatctac aagaagacca gcaaggccgc ggccgcggtc ggagatgagc agaggagcat 1020

ggagtgcgag gactccgtgg aggacgcggt caccgcgtac ccgccctacg ccacggcggg 1080

catggccggc gcaggtgcgc atggcagcaa ctacgttcaa ctgctccatc atcacgacag 1140

ccacgaggac aacttccagc tagacggcct gctcacagaa cacgacgtcg gcctctcggc 1200

gggcgccgcc tcgctgggcc accttgccgc ggcggcgagg gccaccaaac agttcctcgc 1260

cccgtcgtcc tcaaccccgt tcaactggct cgaggcgtca accggtggca gcatcctccc 1320

acaggcaagg aatttccctg ggtttaacag gagcagaaac gtcggcagta tgtcgctgtc 1380

atccacggcc gacgacatgg ctggcgcggt ggacgtcagc gacggaggca atgcggtgaa 1440

cgccatgtat ctccccgtgc aagacgggac ctaccatcag catgtcatcc tcggagctcc 1500

gctggcgcca gaggccatcg cgggcgccgc cacctctggt ttccagcatc acgtccaaat 1560

atccggcgtg aactggaatc cctga 1585

<210> 4
<211> 406
<212> PRT
<213> Hordeum vulgare, haplotype 2

<400> 4

Met Gly Ser Pro Asp Ser Ser Ser Gly Ser Ala Gln Lys Pro Pro Arg
1               5                   10                  15

His Gln His Gln His Gln Pro Pro Pro Pro Arg Arg Gln Gly Ser Ala
            20                  25                  30

Pro Glu Leu Pro Pro Gly Phe Arg Phe His Pro Thr Asp Glu Glu Leu
        35                  40                  45

Val Val His Tyr Leu Lys Lys Lys Ala Ala Lys Ala Pro Leu Pro Val
    50                  55                  60

Thr Ile Ile Ala Glu Val Asp Leu Tyr Lys Phe Asp Pro Trp Glu Leu
65                  70                  75                  80

Pro Glu Lys Ala Thr Phe Gly Glu His Glu Trp Tyr Phe Phe Ser Pro
                85                  90                  95

Arg Asp Arg Lys Tyr Pro Asn Gly Ala Arg Pro Asn Arg Ala Ala Thr
            100                 105                 110

Ser Gly Tyr Trp Lys Ala Thr Gly Thr Asp Lys Pro Ile Leu Ala Ser
        115                 120                 125

Ala Thr Gly Cys Gly Arg Glu Lys Val Gly Val Lys Lys Ala Leu Val
    130                 135                 140

Phe Tyr Arg Gly Lys Pro Pro Arg Gly Leu Lys Thr Asn Trp Ile Met
145                 150                 155                 160

His Glu Tyr Arg Leu Thr Gly Ala Ser Ala Gly Ser Thr Thr Thr Ser
                165                 170                 175

Arg Pro Pro Pro Val Thr Gly Gly Ser Arg Ala Pro Ala Ser Leu Arg
            180                 185                 190

Leu Asp Asp Trp Val Leu Cys Arg Ile Tyr Lys Lys Thr Ser Lys Ala
        195                 200                 205

Ala Ala Ala Val Gly Asp Glu Gln Arg Ser Met Glu Cys Glu Asp Ser
    210                 215                 220

Val Glu Asp Ala Val Thr Ala Tyr Pro Pro Tyr Ala Thr Ala Gly Met
225                 230                 235                 240

Ala Gly Ala Gly Ala His Gly Ser Asn Tyr Val Gln Leu Leu His His
                245                 250                 255

His Asp Ser His Glu Asp Asn Phe Gln Leu Asp Gly Leu Leu Thr Glu
            260                 265                 270

His Asp Val Gly Leu Ser Ala Gly Ala Ala Ser Leu Gly His Leu Ala
        275                 280                 285

Ala Ala Ala Arg Ala Thr Lys Gln Phe Leu Ala Pro Ser Ser Ser Thr
    290                 295                 300

Pro Phe Asn Trp Leu Glu Ala Ser Thr Gly Gly Ser Ile Leu Pro Gln
305                 310                 315                 320

Ala Arg Asn Phe Pro Gly Phe Asn Arg Ser Arg Asn Val Gly Ser Met
                325                 330                 335

Ser Leu Ser Ser Thr Ala Asp Asp Met Ala Gly Ala Val Asp Val Ser
            340                 345                 350

Asp Gly Gly Asn Ala Val Asn Ala Met Tyr Leu Pro Val Gln Asp Gly
        355                 360                 365

Thr Tyr His Gln His Val Ile Leu Gly Ala Pro Leu Ala Pro Glu Ala
    370                 375                 380

Ile Ala Gly Ala Ala Thr Ser Gly Phe Gln His His Val Gln Ile Ser
385                 390                 395                 400

Gly Val Asn Trp Asn Pro
                405